(19) Europäisches Patentamt
European Patent Office
Office européen des brevets

(11) Veröffentlichungsnummer: **0 595 015 A1**

(12) # EUROPÄISCHE PATENTANMELDUNG

(21) Anmeldenummer: **93115090.8**

(22) Anmeldetag: **20.09.93**

(51) Int. Cl.[5]: **C07D 403/04**, C07D 487/14, A01N 43/58

(30) Priorität: **01.10.92 DE 4233028**

(43) Veröffentlichungstag der Anmeldung: **04.05.94 Patentblatt 94/18**

(84) Benannte Vertragsstaaten: **BE CH DE FR GB IT LI NL**

(71) Anmelder: **BAYER AG**
**D-51368 Leverkusen(DE)**

(72) Erfinder: **Seitz, Thomas, Dr.**
**Mozartstrasse 32**
**D-40789 Monheim(DE)**
Erfinder: **Santel, Hans-Joachim, Dr.**
**Grünstrasse 9a**
**D-51371 Leverkusen(DE)**
Erfinder: **Schmidt, Robert R., Dr.**
**Im Waldwinkel 110**
**D-51467 Bergisch Gladbach(DE)**

(54) **Imidazolinyl-pyrimidine und ihre Verwendung als Herbizide.**

(57) Die Erfindung betrifft neue Imidazolinyl-pyrimidine der allgemeinen Formel (I),

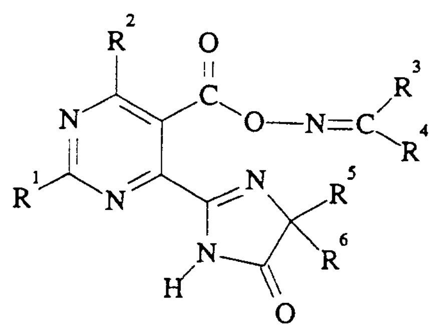

(I)

in welcher

- $R^1$ für Wasserstoff, Halogen, Cyano, Nitro oder Thiocyanato oder für einen jeweils gegebenenfalls substitituierten Hydroxy-, Mercapto-, Amino-, Carbonyl-, Sulfinyl-, Sulfonyl-, Alkyl-, Alkenyl-, Alkinyl-, Cycloalkyl-, Cycloalkenyl-, Aryl- oder Heterocyclylrest steht,
- $R^2$ für Wasserstoff, Hydroxy, Halogen, Alkyl oder Halogenalkyl steht,
- $R^3$ für Wasserstoff oder für Alkyl steht und
- $R^4$ für Alkyl oder für gegebenenfalls substituiertes Aryl steht oder
- $R^3$ und $R^4$ gemeinsam mit dem Kohlenstoffatom, an welches sie gebunden sind für einen Cycloalkylrest stehen und
- $R^5$ und $R^6$ unabhängig voneinander jeweils für Wasserstoff, Alkyl, Halogenalkyl oder Cycloalkyl stehen,

ein dreistufiges Verfahren zu ihrer Herstellung und ihre Verwendung als Herbizide.

EP 0 595 015 A1

Die Erfindung betrifft neue Imidazolinyl-pyrimidine, ein dreistufiges Verfahren zu ihrer Herstellung und ihre Verwendung als Herbizide.

Es ist bekannt, daß bestimmte Imidazolinyl-pyridine wie beispielsweise die Verbindung 2-(4,4-Dimethylimidazolin-5-on-2-yl)-pyridin-3-carbonsäuremethylester herbizide Eigenschaften besitzen (vergl. z.B. EP 41623).

Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Es wurden neue Imidazolinyl-pyrimidine der allgemeinen Formel (I),

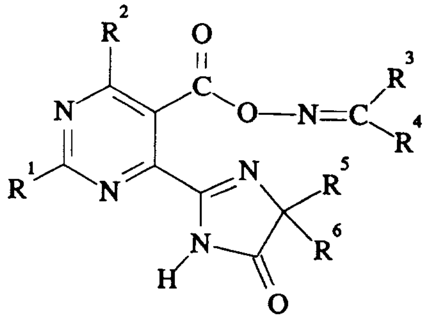

(I)

in welcher

- $R^1$ für Wasserstoff, Halogen, Cyano, Nitro oder Thiocyanato oder für einen jeweils gegebenenfalls substitituierten Hydroxy-, Mercapto-, Amino-, Carbonyl-, Sulfinyl-, Sulfonyl-, Alkyl-, Alkenyl-, Alkinyl-, Cycloalkyl-, Cycloalkenyl-, Aryl- oder Heterocyclylrest steht,
- $R^2$ für Wasserstoff, Hydroxy, Halogen, Alkyl oder Halogenalkyl steht,
- $R^3$ für Wasserstoff oder für Alkyl steht und
- $R^4$ für Alkyl oder für gegebenenfalls substituiertes Aryl steht oder
- $R^3$ und $R^4$ gemeinsam mit dem Kohlenstoffatom, an das sie gebunden sind für einen Cycloalkylrest stehen und
- $R^5$ und $R^6$ unabhängig voneinander jeweils für Wasserstoff, Alkyl, Halogenalkyl oder Cycloalkyl stehen,

gefunden.

Die Verbindungen der Formel (I) können gegebenenfalls in Abhängigkeit von der Art der Substituenten als geometrische und/oder optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen Imidazolinyl-pyrimidine der allgemeinen Formel (I),

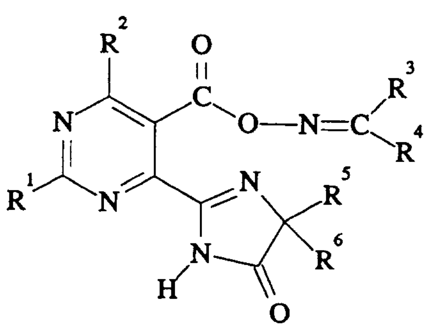

(I)

in welcher

- $R^1$ für Wasserstoff, Halogen, Cyano, Nitro oder Thiocyanato oder für einen jeweils gegebenenfalls substitituierten Hydroxy-, Mercapto-, Amino-, Carbonyl-, Sulfinyl-, Sulfonyl-, Alkyl-, Alkenyl-, Alkinyl-, Cycloalkyl-, Cycloalkenyl-, Aryl- oder Heterocyclylrest steht,
- $R^2$ für Wasserstoff, Hydroxy, Halogen, Alkyl oder Halogenalkyl steht,

| | |
|---|---|
| $R^3$ | für Wasserstoff oder für Alkyl steht und |
| $R^4$ | für Alkyl oder oder für gegebenenfalls substituiertes Aryl steht oder |
| $R^3$ und $R^4$ | gemeinsam mit dem Kohlenstoffatom, an das sie gebunden sind für einen Cycloalkylrest stehen und |
| $R^5$ und $R^6$ | unabhängig voneinander jeweils für Wasserstoff, Alkyl, Halogenalkyl oder Cycloalkyl stehen, |

erhält, wenn man Pyrimidin-dicarbonsäurediester der Formel (II),

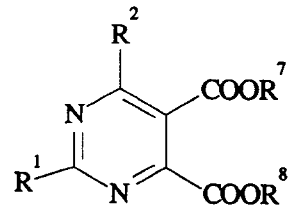

(II)

in welcher

| | |
|---|---|
| $R^1$ und $R^2$ | die oben angegebenen Bedeutungen haben und |
| $R^7$ und $R^8$ | unabhängig voneinander jeweils für Alkyl stehen, |

mit Aminosäureamiden der Formel (III),

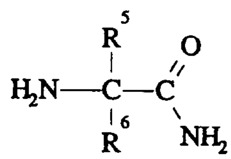

(III)

in welcher

$R^5$ und $R^6$ die oben angegebenen Bedeutungen haben,

zunächst in einer ersten Stufe gegebenenfalls in Gegenwart eines Verdünnungsmittels und gegebenenfalls in Gegenwart eines Reaktionshilfsmittels umsetzt und in einer anschließenden Folgereaktion die so erhältlichen Imidazolinyl-pyrimidin-carbonsäuren der Formel (IV),

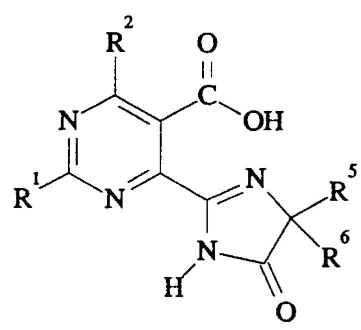

(IV)

in welcher

$R^1$, $R^2$, $R^5$ und $R^6$ die oben angegebenen Bedeutungen haben,

zunächst in einer 2.Stufe mit einem Kondensationsmittel gegebenenfalls in Gegenwart eines Verdünnungsmittels und gegebenenfalls in Gegenwart eines Reaktionshilfsmittels umsetzt und anschließend die so erhältlichen Imidazo-pyrrolo-pyrimidine der Formeln (Va) und (Vb),

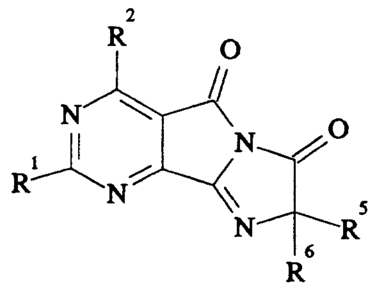

(Va)

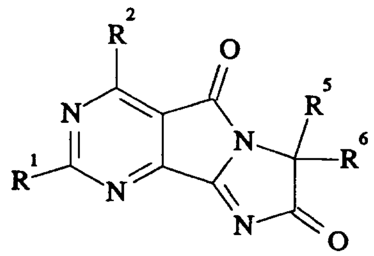

(Vb)

in welchen

$R^1$, $R^2$, $R^5$ und $R^6$ die oben angegebenen Bedeutungen haben,

in einer anschließenden 3. Stufe mit Oximen der Formel (VI),

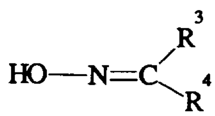

(VI)

in welcher

$R^3$ und $R^4$ die oben angegebenen Bedeutungen haben,

gegebenenfalls in Gegenwart eines Verdünnungsmittels und gegebenenfalls in Gegenwart eines Reaktionshilfsmittels umsetzt.

Schließlich wurde gefunden, daß die neuen Imidazolinyl-pyrimidine der allgemeinen Formel (I) herbizide Eigenschaften besitzen.

Überraschenderweise zeigen die erfindungsgemäßen Imidazolinyl-pyrimidine der allgemeinen Formel (I) eine erheblich bessere herbizide Wirksamkeit gegenüber Problemunkräutern und gleichzeitig eine vergleichbar gute Verträglichkeit gegenüber wichtigen Kulturpflanzen im Vergleich zu den aus dem Stand der Technik bekannten Imidazolinyl-pyridinen, wie beispielsweise die Verbindung 2-(4,4-Dimethylimidazolin-5-on-2-yl)-pyridin-3-carbonsäuremethylester, einer chemisch und wirkungsmäßig naheliegenden Verbindung.

Die erfindungsgemäßen Imidazolinyl-pyrimidine sind durch die Formel (I) allgemein definiert. Bevorzugt sind Verbindungen der Formel (I), bei welchen

$R^1$ für Wasserstoff, Fluor, Chlor, Brom, Iod, Cyano, Nitro, Hydroxy, Mercapto oder Thiocyanato steht;

$R^1$ außerdem für substituiertes -OR oder -SR steht, wobei als Substituenten für R jeweils infrage kommen:

geradkettiges oder verzweigtes Alkyl mit 1 bis 8 Kohlenstoffatomen, geradkettiges oder verzweigtes Halogenalkyl mit 1 bis 8 Kohlenstoffatomen und 1 bis 17 gleichen oder verschiedenen Halogenatomen, geradkettiges oder verzweigtes Alkoxyalkyl mit jeweils 1 bis 8 Kohlenstoffatomen in den einzelnen Alkylteilen, geradkettiges oder verzweigtes Dialkylaminoalkyl mit jeweils 1 bis 8 Kohlenstoffatomen in den einzelnen Alkylteilen, geradkettiges oder verzweigtes Alkanoyl mit 1 bis 9 Kohlenstoffatomen, geradkettiges oder verzweigtes Alkoxycarbonyl mit 2 bis 9 Kohlenstoffatomen, geradkettiges oder verzweigtes Alkylsulfonyl mit 1 bis 8 Kohlenstoffatomen, geradkettiges oder verzweigtes Alkoxysulfonyl mit 1 bis 8 Kohlenstoffatomen, jeweils gegebenenfalls im Aryl- bzw. Heterocyclylteil einfach oder mehrfach, gleich oder verschieden substituiertes Arylalkyl mit 1 bis 8 Kohlenstoffatomen im geradkettigen oder verzweigten Alkylteil und 6 bis 10

Kohlenstoffatomen im Arylteil, Aryl mit 6 bis 10 Kohlenstoffatomen, Arylcarbonyl, Arylsulfonyl oder Aryloxysulfonyl mit jeweils 6 bis 10 Kohlenstoffatomen im Arylteil, Heterocyclyl oder Heterocyclylalkyl mit jeweils 2 bis 9 Kohlenstoffatomen und 1 bis 5 gleichen oder verschiedenen Heteroatomen, insbesondere Stickstoff, Sauerstoff und/oder Schwefel im Heterocyclylteil und gegebenenfalls 1 bis 4 Kohlenstoffatomen im geradkettigen oder verzweigten Alkylteil, wobei als Aryl- bzw. Heterocyclylsubstituenten jeweils infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;

$R^1$ außerdem für gegebenenfalls einfach oder zweifach, gleich oder verschieden substituiertes Amino steht, wobei als Substituenten infrage kommen:

geradkettiges oder verzweigtes Alkyl mit 1 bis 8 Kohlenstoffatomen, geradkettiges oder verzweigtes Alkenyl mit 2 bis 8 Kohlenstoffatomen, geradkettiges oder verzweigtes Alkinyl mit 2 bis 8 Kohlenstoffatomen, geradkettiges oder verzweigtes Halogenalkyl mit 1 bis 8 Kohlenstoffatomen und 1 bis 17 gleichen oder verschiedenen Halogenatomen, geradkettiges oder verzweigtes Alkoxyalkyl mit jeweils 1 bis 8 Kohlenstoffatomen in den einzelnen Alkylteilen, geradkettiges oder verzweigtes Dialkylaminoalkyl mit jeweils 1 bis 8 Kohlenstoffatomen in den einzelnen Alkylteilen, geradkettiges oder verzweigtes Alkanoyl mit 1 bis 9 Kohlenstoffatomen, geradkettiges oder verzweigtes Alkoxycarbonyl mit 2 bis 9 Kohlenstoffatomen, geradkettiges oder verzweigtes Alkylsulfonyl mit 1 bis 8 Kohlenstoffatomen, geradkettiges oder verzweigtes Alkoxysulfonyl mit 1 bis 8 Kohlenstoffatomen, geradkettiges oder verzweigtes Dialkylaminosulfonyl mit jeweils 1 bis 8 Kohlenstoffatomen in den einzelnen Alkylteilen, jeweils gegebenenfalls im Aryl- bzw. Heterocyclylteil einfach oder mehrfach, gleich oder verschieden substituiertes Arylalkyl mit 1 bis 8 Kohlenstoffatomen im geradkettigen oder verzweigten Alkylteil und 6 bis 10 Kohlenstoffatomen im Arylteil, Aryl mit 6 bis 10 Kohlenstoffatomen, Arylcarbonyl ,Arylsulfonyl oder Aryloxysulfonyl mit jeweils 6 bis 10 Kohlenstoffatomen im Arylteil, Heterocyclyl oder Heterocyclylalkyl mit jeweils 2 bis 9 Kohlenstoffatomen und 1 bis 5 gleichen oder verschiedenen Heteroatomen, insbesondere Stickstoff, Sauerstoff und/oder Schwefel im Heterocyclylteil und gegebenenfalls 1 bis 4 Kohlenstoffatomen im geradkettigen oder verzweigten Alkylteil, wobei als Aryl- bzw. Heterocyclylsubstituenten jeweils infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;

$R^1$ außerdem für eine jeweils substituierte Carbonyl-, Sulfinyl- oder Sulfonylgruppe steht, wobei als Substituenten jeweils infrage kommen:

Wasserstoff, Hydroxy, Amino, jeweils geradkettiges oder verzweigtes Alkyl, Alkoxy, Alkylamino oder Dialkylamino mit jeweils 1 bis 8 Kohlenstoffatomen in den einzelnen Alkylteilen, jeweils geradkettiges oder verzweigtes Halogenalkyl oder Halogenalkoxy mit jeweils 1 bis 8 Kohlenstoffatomen und 1 bis 17 gleichen oder verschiedenen Halogenatomen, jeweils gegebenenfalls im Arylteil einfach oder mehrfach, gleich oder verschieden substituiertes Aryl, Aryloxy, Arylamino oder Diarylamino mit jeweils 6 bis 10 Kohlenstoffatomen in den einzelnen Arylteilen, wobei als Substituenten in den einzelnen Arylteilen jeweils infrage kommen:

Halogen, Cyano, Nitro, jeweils geradkettiges oder verzweigtes Akyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9

gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;

$R^1$ außerdem für gegebenenfalls einfach oder mehrfach, gleich oder verschieden substituiertes, geradkettiges oder verzweigtes Alkyl mit 1 bis 8 Kohlenstoffatomen steht, wobei als Substituenten infrage kommen:
Fluor, Chlor, Brom, Iod, Hydroxy, Cyano, Nitro, Aminocarbonyl, Formamido, jeweils geradkettiges oder verzweigtes Alkoxy, Halogenalkoxy, Alkylthio, Halogenalkylthio, Alkylamino, Dialkylamino, Alkanoyl, N-Alkylaminocarbonyl, N,N-Dialkylaminocarbonyl, Alkanoylamido oder Alkylsulfonyl mit jeweils 1 bis 8 Kohlenstoffatomen in den einzelnen Alkylteilen und gegebenenfalls 1 bis 17 gleichen oder verschiedenen Halogenatomen, jeweils gegebenenfalls im Aryl- oder Heterocyclylteil einfach oder mehrfach, gleich oder verschieden substituiertes Aryl, Aryloxy, Arylthio, Arylamino, Diarylamino, Arylcarbonyl oder Arylsulfonyl mit jeweils 6 bis 10 Kohlenstoffatomen in den einzelnen Arylteilen oder Heterocyclyl mit 2 bis 9 Kohlenstoffatomen und 1 bis 5 gleichen oder verschiedenen Heteroatomen, insbesondere Stickstoff, Sauerstoff und/oder Schwefel, wobei als Aryl- oder Heterocyclylsubstituenten jeweils infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;

$R^1$ außerdem für gegebenenfalls einfach oder mehrfach, gleich oder verschieden substituiertes, geradkettiges oder verzweigtes Alkenyl mit 2 bis 8 Kohlenstoffatomen steht, wobei als Substituenten infrage kommen:
Fluor, Chlor, Brom, Iod oder Cyano;

$R^1$ außerdem für gegebenenfalls einfach oder mehrfach, gleich oder verschieden substituiertes, geradkettiges oder verzweigtes Alkinyl mit 2 bis 8 Kohlenstoffatomen steht, wobei als Substituenten infrage kommen:
Fluor, Chlor, Brom oder Iod;

$R^1$ außerdem für jeweils gegebenenfalls einfach oder mehrfach, gleich oder verschieden substituiertes Cycloalkyl oder Cycloalkenyl mit jeweils 3 bis 8 Kohlenstoffatomen steht, wobei als Substituenten jeweils infrage kommen:
Fluor, Chlor, Brom, Iod sowie jeweils geradkettiges oder verzweigtes Alkyl oder Halogenalkyl mit jeweils 1 bis 8 Kohlenstoffatomen und gegebenenfalls 1 bis 17 gleichen oder verschiedenen Halogenatomen;

$R^1$ außerdem für jeweils gegebenenfalls einfach oder mehrfach, gleich oder verschieden substituiertes Aryl mit 6 bis 10 Kohlenstoffatomen oder Heterocyclyl mit 2 bis 9 Kohlenstoffatomen und 1 bis 5 gleichen oder verschiedenen Heteroatomen, insbesondere Stickstoff, Sauerstoff und/oder Schwefel steht, wobei als Aryl- bzw. Heterocyclylsubstituenten jeweils infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;

$R^2$ für Wasserstoff, Hydroxy, Fluor, Chlor, Brom, Iod, für geradkettiges oder verzweigtes Alkyl mit 1 bis 8 Kohlenstoffatomen oder für geradkettiges oder verzweigtes Halogenalkyl mit 1 bis 8 Kohlenstoffatomen und 1 bis 17 gleichen oder verschiedenen Halogenatomen steht,

$R^3$ für Wasserstoff oder für geradkettiges oder verzweigtes Alkyl mit 1 bis 12 Kohlenstoffato-

men steht und

$R^4$ für geradkettiges oder verzweigtes Alkyl mit 1 bis 12 Kohlenstoffatomen oder für gegebenenfalls einfach oder mehrfach, gleich oder verschieden substituiertes Aryl mit 6 bis 10 Kohlenstoffatomen steht, wobei als Arylsubstituenten infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl oder

$R^3$ und $R^4$ gemeinsam mit dem Kohlenstoffatom, an das sie gebunden sind für einen Cycloalkylrest mit 3 bis 8 Kohlenstoffatomen stehen und

$R^5$ und $R^6$ unabhängig voneinander jeweils für Wasserstoff, für geradkettiges oder verzweigtes Alkyl mit 1 bis 8 Kohlenstoffatomen, für geradkettiges oder verzweigtes Halogenalkyl mit 1 bis 8 Kohlenstoffatomen und 1 bis 17 gleichen oder verschiedenen Halogenatomen oder für Cycloalkyl mit 3 bis 8 Kohlenstoffatomen stehen.

Besonders bevorzugt sind Verbindungen der Formel (I), bei welchen

$R^1$ für Wasserstoff, Fluor, Chlor, Brom, Cyano, Nitro, Hydroxy, Mercapto oder Thiocyanato steht;

$R^1$ außerdem für substituiertes -OR oder -SRsteht, wobei als Substituenten für R jeweils infrage kommen:
geradkettiges oder verzweigtes Alkyl mit 1 bis 6 Kohlenstoffatomen, geradkettiges oder verzweigtes Halogenalkyl mit 1 bis 6 Kohlenstoffatomen und 1 bis 13 gleichen oder verschiedenen Halogenatomen, geradkettiges oder verzweigtes Alkoxyalkyl mit jeweils 1 bis 6 Kohlenstoffatomen in den einzelnen Alkylteilen, geradkettiges oder verzweigtes Dialkylaminoalkyl mit jeweils 1 bis 6 Kohlenstoffatomen in den einzelnen Alkylteilen, geradkettiges oder verzweigtes Alkanoyl mit 1 bis 7 Kohlenstoffatomen, geradkettiges oder verzweigtes Alkoxycarbonyl mit 2 bis 7 Kohlenstoffatomen, geradkettiges oder verzweigtes Alkylsulfonyl mit 1 bis 6 Kohlenstoffatomen, geradkettiges oder verzweigtes Alkoxysulfonyl mit 1 bis 6 Kohlenstoffatomen, jeweils gegebenenfalls im Aryl- bzw. Heterocyclylteil einfach bis dreifach, gleich oder verschieden substituiertes Arylalkyl mit 1 bis 6 Kohlenstoffatomen im geradkettigen oder verzweigten Alkylteil und 6 oder 10 Kohlenstoffatomen im Arylteil, Aryl mit 6 oder 10 Kohlenstoffatomen, Arylcarbonyl, Arylsulfonyl oder Aryloxysulfonyl mit jeweils 6 oder 10 Kohlenstoffatomen im Arylteil, Heterocyclyl oder Heterocyclylalkyl mit jeweils 2 bis 9 Kohlenstoffatomen und 1 bis 4 gleichen oder verschiedenen Heteroatomen, insbesondere Stickstoff, Sauerstoff und/oder Schwefel im Heterocyclylteil und gegebenenfalls 1 bis 4 Kohlenstoffatomen im geradkettigen oder verzweigten Alkylteil, wobei als Aryl- bzw. Heterocyclylsubstituenten jeweils infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;

$R^1$ außerdem für gegebenenfalls einfach oder zweifach, gleich oder verschieden substituiertes Amino steht, wobei als Substituenten infrage kommen:
geradkettiges oder verzweigtes Alkyl mit 1 bis 6 Kohlenstoffatomen, geradkettiges oder verzweigtes Alkenyl mit 2 bis 6 Kohlenstoffatomen, geradkettiges oder verzweigtes Alkinyl mit 2 bis 6 Kohlenstoffatomen, geradkettiges oder verzweigtes Halogenalkyl mit 1 bis 6 Kohlenstoffatomen und 1 bis 13 gleichen oder verschiedenen Halogenatomen, geradkettiges oder verzweigtes Alkoxyalkyl mit jeweils 1 bis 6 Kohlenstoffatomen in den einzelnen Alkylteilen, geradkettiges oder verzweigtes Dialkylaminoalkyl mit jeweils 1 bis 6 Kohlenstoffatomen in den einzelnen Alkylteilen, geradkettiges oder verzweigtes Alkanoyl mit 1

bis 7 Kohlenstoffatomen, geradkettiges oder verzweigtes Alkoxycarbonyl mit 2 bis 7 Kohlenstoffatomen, geradkettiges oder verzweigtes Alkylsulfonyl mit 1 bis 6 Kohlenstoffatomen, geradkettiges oder verzweigtes Alkoxysulfonyl mit 1 bis 6 Kohlenstoffatomen, geradkettiges oder verzweigtes Dialkylaminosulfonyl mit jeweils 1 bis 6 Kohlenstoffatomen in den einzelnen Alkylteilen, jeweils gegebenenfalls im Aryl- bzw. Heterocyclylteil einfach bis dreifach, gleich oder verschieden substituiertes Arylalkyl mit 1 bis 6 Kohlenstoffatomen im geradkettigen oder verzweigten Alkylteil und 6 oder 10 Kohlenstoffatomen im Arylteil, Aryl mit 6 bis 10 Kohlenstoffatomen, Arylcarbonyl ,Arylsulfonyl oder Aryloxysulfonyl mit jeweils 6 oder 10 Kohlenstoffatomen im Arylteil, Heterocyclyl oder Heterocyclylalkyl mit jeweils 2 bis 9 Kohlenstoffatomen und 1 bis 4 gleichen oder verschiedenen Heteroatomen, insbesondere Stickstoff, Sauerstoff und/oder Schwefel im Heterocyclylteil und gegebenenfalls 1 bis 4 Kohlenstoffatomen im geradkettigen oder verzweigten Alkylteil, wobei als Aryl- bzw. Heterocyclylsubstituenten jeweils infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;

$R^1$ außerdem für eine jeweils substituierte Carbonyl-, Sulfinyl- oder Sulfonylgruppe steht, wobei als Substituenten jeweils infrage kommen:

Wasserstoff, Hydroxy, Amino, jeweils geradkettiges oder verzweigtes Alkyl, Alkoxy, Alkylamino oder Dialkylamino mit jeweils 1 bis 6 Kohlenstoffatomen in den einzelnen Alkylteilen, jeweils geradkettiges oder verzweigtes Halogenalkyl oder Halogenalkoxy mit jeweils 1 bis 6 Kohlenstoffatomen und 1 bis 13 gleichen oder verschiedenen Halogenatomen, jeweils gegebenenfalls im Arylteil einfach bis dreifach. gleich oder verschieden substituiertes Aryl, Aryloxy, Arylamino oder Diarylamino mit jeweils 6 oder 10 Kohlenstoffatomen in den einzelnen Arylteilen, wobei als Substituenten in den einzelnen Arylteilen jeweils infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;

$R^1$ außerdem für gegebenenfalls einfach bis fünffach, gleich oder verschieden substituiertes, geradkettiges oder verzweigtes Alkyl mit 1 bis 6 Kohlenstoffatomen steht, wobei als Substituenten infrage kommen:

Fluor, Chlor, Brom, Iod, Hydroxy, Cyano, Nitro, Aminocarbonyl, Formamido, jeweils geradkettiges oder verzweigtes Alkoxy, Halogenalkoxy, Alkylthio, Halogenalkylthio, Alkylamino, Dialkylamino, Alkanoyl, N-Alkylaminocarbonyl, N,N-Dialkylaminocarbonyl, Alkanoylamido oder Alkylsulfonyl mit jeweils 1 bis 6 Kohlenstoffatomen in den einzelnen Alkylteilen und gegebenenfalls 1 bis 13 gleichen oder verschiedenen Halogenatomen, jeweils gegebenenfalls im Aryl- oder Heterocyclylteil einfach bis dreifach, gleich oder verschieden substituiertes Aryl, Aryloxy, Arylthio, Arylamino, Diarylamino, Arylcarbonyl oder Arylsulfonyl mit jeweils 6 oder 10 Kohlenstoffatomen in den einzelnen Arylteilen oder Heterocyclyl mit 2 bis 9 Kohlenstoffatomen und 1 bis 4 gleichen oder verschiedenen Heteroatomen, insbesondere Stickstoff, Sauerstoff und/oder Schwefel, wobei als Aryl- oder Heterocyclylsubstituenten jeweils infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;

$R^1$ außerdem für gegebenenfalls einfach bis fünffach, gleich oder verschieden substituiertes, geradkettiges oder verzweigtes Alkenyl mit 2 bis 6 Kohlenstoffatomen steht, wobei als Substituenten infrage kommen:
Fluor, Chlor, Brom oder Cyano;

$R^1$ außerdem für gegebenenfalls einfach bis dreifach, gleich oder verschieden substituiertes, geradkettiges oder verzweigtes Alkinyl mit 2 bis 6 Kohlenstoffatomen steht, wobei als Substituenten infrage kommen:
Fluor, Chlor oder Brom;

$R^1$ außerdem für jeweils gegebenenfalls einfach bis fünffach, gleich oder verschieden substituiertes Cycloalkyl oder Cycloalkenyl mit jeweils 3 bis 7 Kohlenstoffatomen steht, wobei als Substituenten jeweils infrage kommen:
Fluor, Chlor, Brom, Iod sowie jeweils geradkettiges oder verzweigtes Alkyl oder Halogenalkyl mit jeweils 1 bis 6 Kohlenstoffatomen und gegebenenfalls 1 bis 13 gleichen oder verschiedenen Halogenatomen;

$R^1$ außerdem für jeweils gegebenenfalls einfach bis fünffach, gleich oder verschieden substituiertes Aryl mit 6 oder 10 Kohlenstoffatomen oder Heterocyclyl mit 2 bis 9 Kohlenstoffatomen und 1 bis 4 gleichen oder verschiedenen Heteroatomen, insbesondere Stickstoff, Sauerstoff und/oder Schwefel steht, wobei als Aryl- bzw. Heterocyclylsubstituenten jeweils infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;

$R^2$ für Wasserstoff, Hydroxy, Fluor, Chlor, Brom, Iod, für geradkettiges oder verzweigtes Alkyl mit 1 bis 6 Kohlenstoffatomen oder für geradkettiges oder verzweigtes Halogenalkyl mit 1 bis 6 Kohlenstoffatomen und 1 bis 13 gleichen oder verschiedenen Halogenatomen steht,
$R^3$ für Wasserstoff oder für geradkettiges oder verzweigtes Alkyl mit 1 bis 8 Kohlenstoffatomen steht und

$R^4$ für geradkettiges oder verzweigtes Alkyl mit 1 bis 8 Kohlenstoffatomen oder für gegebenenfalls einfach bis fünffach, gleich oder verschieden substituiertes Aryl mit 6 oder 10 Kohlenstoffatomen steht, wobei als Arylsubstituenten infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl oder

$R^3$ und $R^4$ gemeinsam mit dem Kohlenstoffatom, an das sie gebunden sind für einen Cycloalkylrest mit 3 bis 7 Kohlenstoffatomen stehen und

$R^5$ und $R^6$ unabhängig voneinander jeweils für Wasserstoff, für geradkettiges oder verzweigtes Alkyl mit 1 bis 6 Kohlenstoffatomen, für geradkettiges oder verzweigtes Halogenalkyl mit 1 bis 6 Kohlenstoffatomen und 1 bis 13 gleichen oder verschiedenen Halogenatomen oder für Cycloalkyl mit 3 bis 7 Kohlenstoffatomen stehen.

Insbesondere bevorzugt sind Verbindungen der Formel (I), bei welchen

$R^1$ für Wasserstoff, Hydroxy oder Mercapto steht;

$R^1$ außerdem für substituiertes -OR oder -SR steht, wobei als Substituenten für R jeweils infrage kommen:
geradkettiges oder verzweigtes Alkyl mit 1 bis 4 Kohlenstoffatomen, geradkettiges oder verzweigtes Alkoxyalkyl mit jeweils 1 bis 4 Kohlenstoffatomen in den einzelnen Alkylteilen,

geradkettiges oder verzweigtes Dialkylaminoalkyl mit jeweils 1 bis 4 Kohlenstoffatomen in den einzelnen Alkylteilen, geradkettiges oder verzweigtes Halogenalkyl mit 1 bis 4 Kohlenstoffatomen und 1 bis 9 gleichen oder verschiedenen Halogenatomen oder jeweils gegebenenfalls im Phenyl- bzw. Heteroarylteil einfach bis dreifach, gleich oder verschieden substituiertes Phenylalkyl mit 1 bis 4 Kohlenstoffatomen im geradkettigen oder verzweigten Alkylteil,

Phenyl, Pyridyl, Pyridylmethyl, Furanylmethyl, Thienylmethyl, Thiazolylmethyl oder Triazolylmethyl, wobei als Phenyl- bzw. Heteroarylsubstituenten jeweils infrage kommen:

Fluor, Chlor, Brom, Iod, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen - insbesondere Fluor, Chlor, Brom und/oder Iod;

$R^1$ außerdem für gegebenenfalls einfach oder zweifach, gleich oder verschieden substituiertes Amino steht, wobei als Substituenten infrage kommen:

geradkettiges oder verzweigtes Alkyl mit 1 bis 4 Kohlenstoffatomen, geradkettiges oder verzweigtes Alkenyl mit 2 bis 6 Kohlenstoffatomen, Allyl, Propargyl, geradkettiges oder verzweigtes Alkoxyalkyl mit jeweils 1 bis 4 Kohlenstoffatomen in den einzelnen Alkylteilen, geradkettiges oder verzweigtes Dialkylaminoalkyl mit jeweils 1 bis 4 Kohlenstoffatomen in den einzelnen Alkylteilen, geradkettiges oder verzweigtes Alkanoyl mit 1 bis 5 Kohlenstoffatomen, geradkettiges oder verzweigtes Alkoxycarbonyl mit 2 bis 5 Kohlenstoffatomen, geradkettiges oder verzweigtes Alkylsulfonyl mit 1 bis 4 Kohlenstoffatomen;

$R^1$ außerdem für eine jeweils substituierte Carbonyl-, Sulfinyl- oder Sulfonylgruppe steht, wobei als Substituenten jeweils infrage kommen:

jeweils geradkettiges oder verzweigtes Alkyl, Alkoxy, Alkylamino oder Dialkylamino mit jeweils 1 bis 4 Kohlenstoffatomen in den einzelnen Alkylteilen;

$R^1$ außerdem für gegebenenfalls einfach bis fünffach, gleich oder verschieden substituiertes, geradkettiges oder verzweigtes Alkyl mit 1 bis 6 Kohlenstoffatomen steht, wobei als Substituenten infrage kommen:

Fluor, Chlor, jeweils geradkettiges oder verzweigtes Alkoxy oder Alkylthio mit jeweils 1 bis 4 Kohlenstoffatomen; geradkettiges oder verzweigtes Halogenalkoxy oder Halogenalkylthio mit jeweils 1 bis 4 Kohlenstoffatomen und 1 bis 9 gleichen oder verschiedenen Halogenatomen - insbesondere Fluor, Chlor und/oder Brom - oder gegebenenfalls einfach bis dreifach, gleich oder verschieden substituiertes Phenyl, wobei als Substituenten jeweils infrage kommen:

Halogen, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen;

$R^1$ außerdem für geradkettiges oder verzweigtes Alkenyl mit 2 bis 6 Kohlenstoffatomen steht;

$R^1$ außerdem für geradkettiges oder verzweigtes Alkinyl mit 2 bis 6 Kohlenstoffatomen steht;

$R^1$ außerdem für gegebenenfalls einfach bis dreifach, gleich oder verschieden durch Methyl und/oder Chlor substituiertes Cyclopropyl steht;

$R^1$ außerdem für gegebenenfalls einfach bis dreifach, gleich oder verschieden substituiertes Phenyl steht, wobei als Substituenten jeweils infrage kommen:

Fluor, Chlor, Brom, Iod, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen - insbesondere Fluor, Chlor, Brom und/oder Iod;

$R^1$ außerdem für jeweils gegebenenfalls einfach bis dreifach, gleich oder verschieden substituiertes Pyridyl, Furanyl, Tetrahydrofuranyl, Thienyl oder Thiazolyl steht, wobei als Substituenten jeweils infrage kommen:

jeweils geradkettiges oder verzweigtes Alkyl oder Alkoxy mit jeweils 1 bis 4 Kohlenstoffatomen;

$R^2$ für Wasserstoff oder Methyl steht;

$R^3$ für Wasserstoff oder für geradkettiges oder verzweigtes Alkyl mit 1 bis 6 Kohlenstoffatomen steht und

$R^4$ für geradkettiges oder verzweigtes Alkyl mit 1 bis 6 Kohlenstoffatomen oder für gegebenenfalls einfach bis dreifach, gleich oder verschieden substituiertes Phenyl steht, wobei als Phenylsubstituenten infrage kommen:
Fluor, Chlor, Brom, Iod, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen - insbesondere Fluor, Chlor, Brom und/oder Iod oder

$R^3$ und $R^4$ gemeinsam mit dem Kohlenstoffatom, an das sie gebunden sind für einen Cycloalkylrest mit 3 bis 6 Kohlenstoffatomen stehen,

$R^5$ für Wasserstoff, Methyl oder Fluormethyl steht und

$R^6$ für Methyl, Ethyl, n- oder i-Propyl oder Cyclopropyl steht.

Ganz besonders bevorzugt sind Verbindungen der Formel (I), bei welchen

$R^1$ für Wasserstoff, Hydroxy, Methoxy, Ethoxy, n- oder i-Propoxy, n-, i-, s- oder t-Butoxy, Trifluormethoxy, Mercapto, Methylthio, Ethylthio, n- oder i-Propylthio, n-, i-, s- oder t-Butylthio, Trifluormethylthio, Benzylthio, 2-Pyridylthio, Amino, N-Methylamino, N-Ethylamino, N-n-Propylamino, N-i-Propylamino, N,N-Dimethylamino, N,N-Diethylamino, N-Methyl-N-Ethylamino, Methylcarbonylamino, Ethylcarbonylamino, Methoxycarbonylamino, Ethoxycarbonylamino, Methylsulfinyl, Ethylsulfinyl, Methylsulfonyl, Ethylsulfonyl, Methyl, Ethyl, n- oder i-Propyl, n-, i-, s- oder t-Butyl, n- oder i-Pentyl, n- oder i-Hexyl, Fluormethyl, Trifluormethyl, Methoxymethyl, Ethoxymethyl, Methylthiomethyl, Ethylthiomethyl, Allyl, Ethinyl, Propargyl, Cyclopropyl oder für gegebenenfalls einfach oder zweifach, gleich oder verschieden substituiertes Phenyl steht, wobei als Substituenten infrage kommen:
Fluor, Chlor, Brom, Methyl, Ethyl, n- oder i-Propyl, n-, i-, s- oder t-Butyl, Methoxy, Ethoxy, n- oder i-Propoxy, n-, i-,s- oder t-Butoxy, Methylthio, Ethylthio, Trifluormethyl, Trifluormethoxy oder Trifluormethylthio;

$R^1$ außerdem für jeweils gegebenenfalls einfach oder zweifach, gleich oder verschieden substituiertes Pyridyl, Furanyl, Thienyl oder Thiazolyl steht, wobei als Substituenten jeweils infrage kommen:
Methyl, Ethyl, n- oder i-Propyl, n-, i-, s- oder t-Butyl, Methoxy, Ethoxy, n- oder i-Propoxy, n-, i-, s- oder t-Butoxy,

$R^2$ für Wasserstoff steht,

$R^3$ für Wasserstoff oder für geradkettiges oder verzweigtes Alkyl mit 1 bis 4 Kohlenstoffatomen steht und

$R^4$ für geradkettiges oder verzweigtes Alkyl mit 1 bis 4 Kohlenstoffatomen oder für gegebenenfalls einfach bis dreifach, gleich oder verschieden substituiertes Phenyl steht, wobei als Phenylsubstituenten infrage kommen:
Fluor, Chlor, Brom, Methyl, Ethyl, n- oder i-Propyl, n-, i-, s- oder t-Butyl, Methoxy, Ethoxy, n- oder i-Propoxy, n-, i-,s- oder t-Butoxy, Methylthio, Ethylthio, Trifluormethyl, Trifluormethoxy oder Trifluormethylthio oder

$R^3$ und $R^4$ gemeinsam mit dem Kohlenstoffatom, an welches sie gebunden sind für einen Cyclopropyl-, Cyclopentyl oder Cyclohexylrest stehen,

$R^5$ für Methyl steht und

$R^6$ für Isopropyl steht.

Im einzelnen seien die bei den Herstellungsbeispielen genannten Verbindungen der allgemeinen Formel (I) genannt.

Verwendet man beispielsweise 2-Methyl-pyrimidin-5,6-dicarbonsäurediethylester und 2-Amino-2,3-dimethylbuttersäureamid als Ausgangsverbindungen, so läßt sich der Reaktionsablauf der 1. Stufe des erfindungsgemäßen Verfahrens durch das folgende Formelschema darstellen:

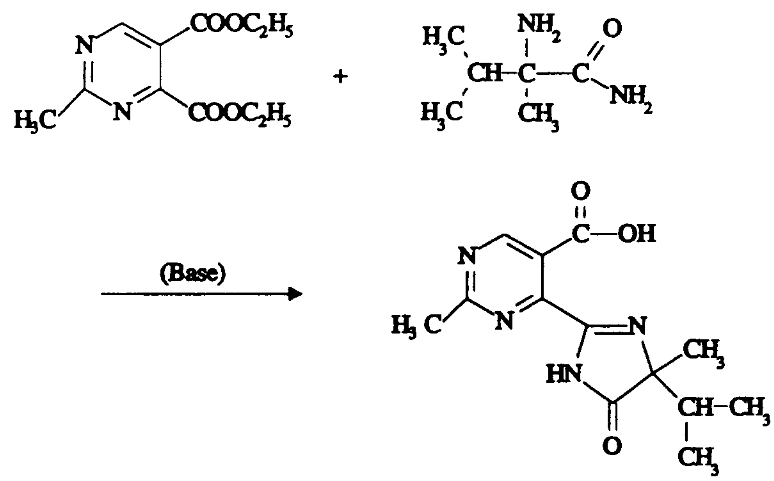


Verwendet man beispielsweise 2-Methyl-4-(4-methyl-4-isopropylimidazolin-5-on-2-yl)-pyrimidin-5-carbonsäure und Butanonoxim als Ausgangsstoffe sowie N,N'-Dicyclohexylcarbodiimid als Kondensationsmittel, so läßt sich der Reaktionsablauf der 2. und 3. Stufe des erfindungsgemäßen Verfahrens durch das folgende Formelschema darstellen:

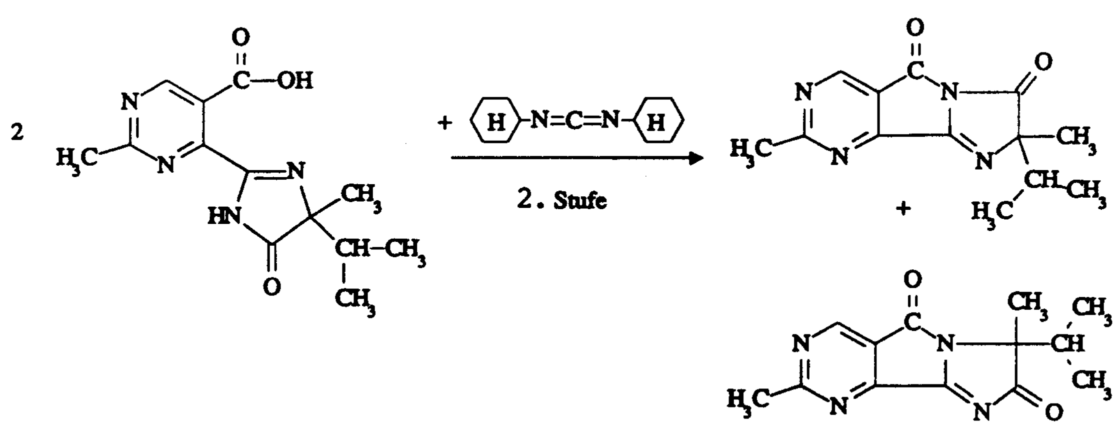


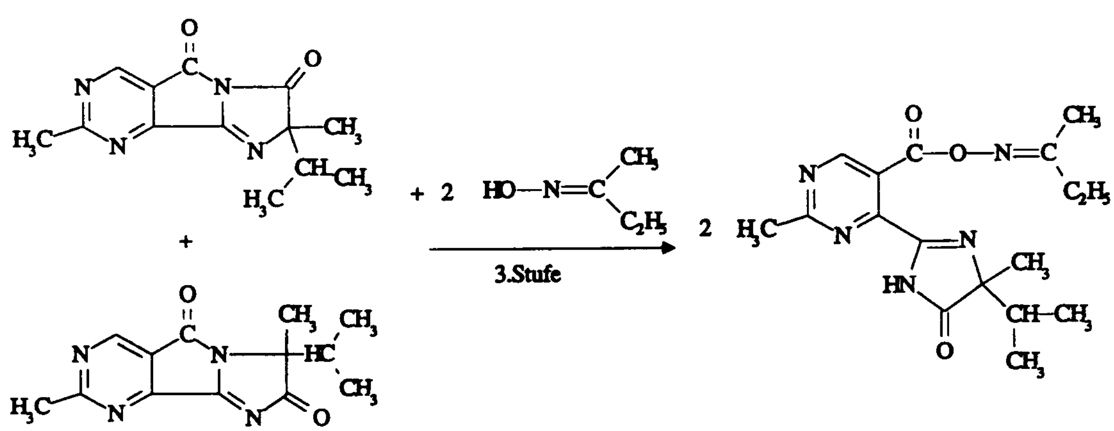


Die zur Durchführung des erfindungsgemäßen Verfahrens als Ausgangsstoffe benötigten Pyrimidin-dicarbonsäurediester sind durch die Formel (II) allgemein definiert. In dieser Formel (II) stehen $R^1$ und $R^2$ vorzugsweise für diejenigen Reste, die bereits im Zusammenhang mit der Beschreibung der erfindungsgemäßen Verbindungen der Formel (I) als bevorzugt für diese Substituenten genannt wurden. $R^6$ und $R^7$ stehen vorzugsweise unabhängig voneinander jeweils für geradkettiges oder verzweigtes Alkyl mit 1 bis 4

Kohlenstoffatomen, insbesondere für Methyl oder Ethyl.

Die Pyrimidin-dicarbonsäurediester der Formel (II) sind bekannt oder erhältlich in Analogie zubekannten Verfahren (vergl. z.B. EP 305 184; J. Chem Soc. , Perkin Trans 1, 1980, 1667-1670; Justus Liebigs Ann. Chem. 1977, 1413-1420; J. Heterocycl. Chem. 14, 695-696 [1977]; Bull. Soc. Chim. Fr. 9-10 Pt.2, 1543-1548, [1976]; Justus Liebigs Ann. Chem. 1976, 1809-1819; Chem. Ber. 108, 3877-3882 [1975]; Arch. Pharm. 308, 118-121 [1975]; JP 49024077; Justus Liebigs Ann. Chem. 1974, 1190-1194; DE 22 42 162; Chem. Pharm. Bull. 20, 1513-1521 [1972]; DE 20 46 577; J. Amer. Chem. Soc. 84, 837-844 [1962]; J. Heterocycl. Chem. 2, 202-204 [1965]; Chem. Pharm. Bull. 8, 262-264 [1960]; J. Org. Chem. 20, 1342-1346 [1955]; US 2.774.760).

Die zur Durchführung des erfindungsgemäßen Verfahrens weiterhin als Ausgangsstoffe erforderlichen Aminosäureamide sind durch die Formel (III) allgemein definiert. In dieser Formel (III) stehen $R^5$ und $R^6$ vorzugsweise für diejenigen Reste, die bereits im Zusammenhang mit der Beschreibung der erfindungsgemäßen Stoffe der Formel (I) als bevorzugt für diese Substituenten genannt wurden.

Die Aminosäureamide der Formel (III) sind allgemein bekannt oder erhältlich in Analogie zu allgemein bekannten Verfahren (vergl. z.B. Houben-Weyl-Müller "Methoden der organischen Chemie", Thieme Verlag Stuttgart 1974; Band XV/1, S.46ff; Band XV/2, S.112ff).

Die zur Durchführung des erfindungsgemäßen Verfahrens weiterhin als Ausgangsstoffe erforderlichen Oxime sind durch die Formel (VI) allgemein definiert. In dieser Formel (VI) stehen $R^3$ und $R^4$ vorzugsweise für diejenigen Reste, die bereits im Zusammenhang mit der Beschreibung der erfindungsgemäßen Stoffe der Formel (I) als bevorzugt für diese Substituenten genannt wurden.

Die Oxime der Formel (VI) sind allgemein bekannte Verbindungen der organischen Chemie.

Als Verdünnungsmittel zur Durchführung der 1. Stufe des erfindungsgemäßen Verfahrens kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform oder Tetrachlorkohlenstoff; Ether, wie Diethylether, Diisopropylether, Dioxan, Tetrahydrofuran oder Ethylenglykoldimethyl- oder -diethylether oder Amide, wie N,N-Dimethylformamid, N,N-Dimethylacetamid, N-Methylformanilid, N-Methylpyrrolidon oder Hexamethylphosphorsäuretriamid.

Die 1. Stufe des erfindungsgemäßen Verfahrens wird vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumcetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin, Piperidin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung der 1. Stufe des erfindungsgemäßen Verfahrens in einem größeren Bereich variiert werden. Im allgemeinen arbeitet man bei Temperaturen zwischen 0°C und 180°C, vorzugsweise bei Temperaturen zwischen 20°C und 120°C.

Die 1. Stufe des erfindungsgemäßen Verfahrens wird üblicherweise unter Normaldruck durchgeführt. Es ist jedoch auch möglich unter erhöhtem oder vermindertem Druck zu arbeiten.

Zur Durchführung der 1. Stufe des erfindungsgemäßen Verfahrens setzt man pro Mol Pyrimidindicarbonsäurediester der Formel (II) im allgemeinen 1,0 bis 1,5 Mol, vorzugsweise äquimolare Mengen Aminosäureamid der Formel (III) und 1,0 bis 5,0 Mol, vorzugsweise 1,0 bis 3,0 Mol Base als Reaktionshilfsmittel ein. Die Reaktionsdurchführung, Aufarbeitung und Isolierung der Reaktionsprodukte erfolgt nach bekannten Verfahren (vergl. hierzu beispielsweise EP 41623 oder die Herstellungsbeispiele).

Die 2. Stufe des erfindungsgemäßen Verfahrens wird in Gegenwart eines geeigneten Kondensationsmittels durchgeführt. Als solche kommen alle für derartige Cyclisierungsreaktionen üblichen Kondensationsmittel infrage. Beispielhaft genannt seien Säurehalogenidbildner, wie Phosphortribromid, Phosphorpentachlorid, Phosphoroxychlorid oder Thionylchlorid, Anhydridbildner, wie Chlorameisensäureethylester oder Methansulfonylchlorid, Carbodiimide, wie N,N'-Dicyclohexylcarbodiimid (DCC) oder N,N'-Diisopropylcarbodiimid oder andere übliche Kondensationsmittel, wie N,N'-Carbonyldiimidazol, Triphenylphosphin/Tetrachlorkohlenstoff oder 2-Ethoxy-N-ethoxycarbonyl-1,2-dihydrochinolin (EEDQ).

Als Verdünnungsmittel zur Durchführung der 2. Stufe des erfindungsgemäßen Verfahrens kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester oder Sulfoxide, wie Dimethylsulfoxid oder Alkohole, wie Methanol, Ethanol oder Isopropanol.

Die 2. Stufe des erfindungsgemäßen Verfahrens kann gegebenenfalls in Gegenwart eines geeigneten Reaktionshilfsmittels durchgeführt werden. Als solche kommen alle üblichen anorganischen oder organischen Basen infrage. Hierzu gehören beispielsweise Erdalkali- oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin, Piperidin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung der 2. Stufe des erfindungsgemäßen Verfahrens in einem größeren Bereich variiert werden. Im allgemeinen arbeitet man bei Temperaturen zwischen -20°C und 100°C, vorzugsweise bei Temperaturen zwischen 0°C und 80°C.

Zur Durchführung der 2. Stufe des erfindungsgemäßen Verfahrens setzt man pro Mol Imidazolinyl-pyrimidin-carbonsäure der Formel (IV) im allgemeinen 1,0 bis 5,0 Mol, vorzugsweise 1,0 bis 1,5 Mol Kondensationsmittel und gegebenenfalls 0,001 bis 2,0 Mol, vorzugsweise 0,1 bis 1,2 Mol Base als Reaktionshilfsmittel ein. Die Reaktionsdurchführung, Aufarbeitung und Isolierung der Reaktionsprodukte erfolgt nach bekannten Verfahren (vergl. hierzu beispielsweise EP 41623 oder die Herstellungsbeispiele).

Als Verdünnungsmittel zur Durchführung der 3. Stufe des erfindungsgemäßen Verfahrens kommen ebenfalls inerte organische Lösungsmittel infrage. Vorzugsweise verwendet man die bei der 2. Stufe des erfindungsgemäßen Verfahrens genannten Verdünnungsmittel.

Die 3. Stufe des erfindungsgemäßen Verfahrens kann gegebenenfalls in Gegenwart eines geeigneten basischen Reaktionshilfsmittels durchgeführt werden. Als solche kommen alle üblichen anorganischen oder organischen Basen infrage. Hierzu gehören beispielsweise Erdalkali- oder Alkälimetallhydroxide, wie Natriumhydroxid, Calciumhydroxid, Kaliumhydroxid oder auch Ammoniumhydroxid, Alkalimetallcarbonate, wie Natriumcarbonat, Kaliumcarbonat, Kaliumhydrogencarbonat, Natriumhydrogencarbonat oder Ammoniumcarbonat, Alkali- oder Erdalkalimetallacetate, wie Natriumacetat, Kaliumacetat, Calciumacetat oder Ammoniumacetat, sowie tertiäre Amine, wie Trimethylamin, Triethylamin, Tributylamin, N,N-Dimethylanilin, Pyridin, Piperidin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung der 3. Stufe des erfindungsgemäßen Verfahrens in einem größeren Bereich variiert werden. Im allgemeinen arbeitet man bei Temperaturen zwischen 0°C und 150°C, vorzugsweise bei Temperaturen zwischen 20°C und 100°C.

Die 3. Stufe des erfindungsgemäßen Verfahrens wird üblicherweise unter Normaldruck durchgeführt.

Zur Durchführung der 3. Stufe des erfindungsgemäßen Verfahrens setzt man pro Mol Imidazo-pyrrolo-pyrimidin der Formeln (Va) bzw. (Vb) im allgemeinen 1,0 bis 15,0 Mol, vorzugsweise 1,0 bis 5,0 Mol Oxim der Formel (VI) und gegebenenfalls 0,1 bis 2,0 Mol, vorzugsweise 0,5 bis 1,2 Mol Base als Reaktionshilfsmittel ein.

Die Reaktionsdurchführung, Aufarbeitung und Isolierung der Reaktionsprodukte erfolgt nach bekannten Verfahren (vergl. hierzu beispielsweise EP 41623 oder die Herstellungsbeispiele).

In einer besonderen Durchführungsform ist es auch möglich, die 2. und die 3. Stufe des erfindungsgemäßen Verfahrens in einem Reaktionschritt in einem sogenannten "Eintopfverfahren" durchzuführen. Man geht dabei von Imidazolinyl-pyrimidin-carbonsäure der Formel (IV) aus und setzt diese nacheinander im "Eintopfverfahren" zunächst mit einem Kondensationsmittel und anschließend mit einem Alkohol oder Thiol der Formel (VI) um.

Die Reinigung der Endprodukte der Formel (I) erfolgt mit Hilfe üblicher Verfahren, beispielsweise durch Säulenchromatographie oder durch Umkristallisieren.

Die Charakterisierung der Verbindungen erfolgt mit Hilfe des Schmelzpunktes oder bei nicht kristallisierenden Verbindungen mit Hilfe der Protonen-Kernresonanzspektroskopie ($^1$H-NMR).

Die erfindungsgemäßen Wirkstoffe können als Defoliants, Desiccants, Krautabtötungsmittel und insbesondere als Unkrautvernichtungsmittel verwendet werden.Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewandten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Zitrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur Bekämpfung von monokotylen und dikotylen Unkräutern in mono- und dikotylen Kulturen wie beispielsweise Soja, Baumwolle oder Weizen einsetzen.

Daneben weisen die erfindungsgemäßen Wirkstoffe in entsprechenden Aufwandmengen auch eine fungizide Wirkung auf und können daher auch zur Bekämpfung von Pflanzenkrankheiten, wie beispielsweise gegen den Erreger der Reisfleckenkrankheit (Pyricularia oryzae) eingesetzt werden.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe, wie Chlorbenzole, Chlorethylene, oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid oder Dimethylsulfoxid, sowie Wasser.

Als feste Trägerstoffe kommen infrage: z.B. Ammoniumsalze und natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nicht ionogene und anionische Emulgatoren, wie Polyoxyethylen-Fettsäureester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Ligninsulfitablaugen und Methylcellulose.

Es können in den Formulierungen Haftmittel wie Carboxymethylcellulose, natürliche und synthetische, pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba oder Picloram; Aryloxyalkansäuren, wie z.B. 2,4-D, 2,4-DB, 2,4-DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen; Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro Hektar.

Die Herstellung und die Verwendung der erfindungsgemäßen Wirkstoffe geht aus den nachfolgenden Beispielen hervor.

Herstellungsbeispiele:

Beispiel 1:

1.Stufe (IV-1):

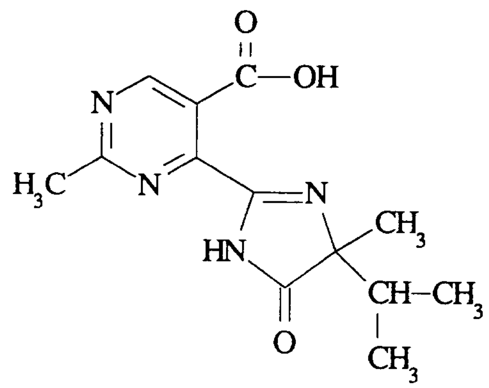

Zu einer Mischung von 98,8 g (0,415 Mol) 2-Methylpyrimidin-4,5-dicarbonsäurediethylester (vergl. z.B. J. Heterocycl. Chem. 2, 202-204 [1965]) und 54,0 g (0,415 Mol) 2-Amino-2,3-dimethylbuttersäureamid in 700 ml wasserfreiem Toluol gibt man portionsweise 102,2 g (0,913 Mol) Kalium-tert.-butylat und rührt anschließend 16 Stunden bei 80°C. Zur Aufarbeitung wird die Reaktionsmischung abgekühlt, der ausgefallene Feststoff abfiltriert, mehrfach mit Diethylether nachgewaschen und anschließend in Wasser gelöst. Die wässrige Lösung wird mit halbkonzentrierter Salzsäure auf pH 5 gebracht und fünfmal mit jeweils 200 ml Dichlormethan extrahiert. Die vereinigten organischen Extrakte werden über Natriumsulfat getrocknet und im Vakuum eingeengt. Der Rückstand läßt sich durch Chromatographie an Kieselgel reinigen.

Man erhält 59 g (51 % der Theorie) 2-Methyl-4-(4-methyl-4-isopropyl-imidazolin-5-on-2-yl)-pyrimidin-5-carbonsäure mit Schmelzpunkt 56-57°C.

2. Stufe (Va/Vb):

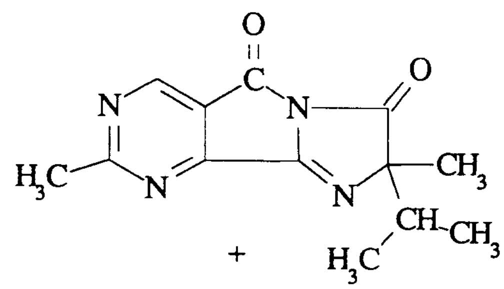

+

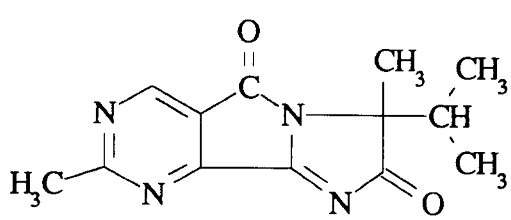

22,8 g (0,082 Mol) 2-Methyl-4-(4-methyl-4-isopropyl-imidazolin-5-on-2-yl)-pyrimidin-5-carbonsäure werden in 150 ml Tetrahydrofuran suspendiert, mit 17,0 g (0,082 Mol) N,N'-Dicyclohexylcarbodiimid versetzt, eine Stunde bei 40°C gerührt, anschließend im Vakuum eingeengt und der Rückstand chromatographisch an Kieselgel gereinigt (Laufmittel: Essigester/Cyclohexan 1:1).

Man erhält 16,4 g (77 % der Theorie) einer Isomerenmischung des oben angegebenen Imidazo-pyrrolo-pyrimidins als Öl.

$^1$H-NMR ($CDCl_3$/Tetramethylsilan): $\delta$ = 0,95 (d,3H); 1,15 (d,3H); 1,58 (s,3H); 1,9 (m,1H); 3,02 (s,3H); 9,32 (s,1H) ppm

3. Stufe (I-1):

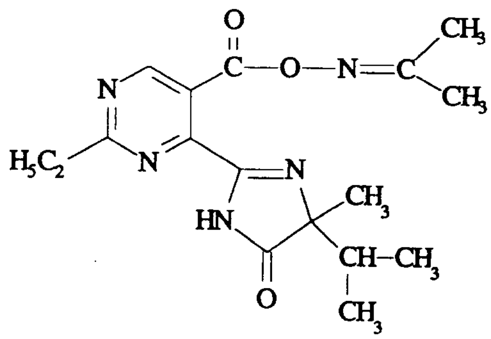

Zu 3,46 g (0,001 Mol) der oben genannten Imidazo-pyrrolo-pyrimidin-Mischung in 100 ml Tetrahydrofuran gibt man nacheinander 3 Tropfen Triethylamin und 0,73 g (0,001 Mol) Acetonoxim und rührt die Mischung anschließend 36 Stunden bei 65 °C. Zur Aufarbeitung wird das Lösungsmittel abdestilliert, der Rückstand in Essigester aufgenommen, mit verdünnter wässriger Salzsäure auf pH 4 angesäuert, die organische Phase abgetrennt, mit Wasser neutral gewaschen, über Natriumsulfat getrocknet und im Vakuum eingeengt. Der Rückstand wird durch Chromatographie an Kieselgel (Laufmittel: Essigester/Hexan 1:3) gereinigt.

Man erhält 3,1 g (60 % der Theorie) 2-Methyl-4-(4-methyl-4-isopropyl-imidazolin-5-on-2-yl)-5-(2-propylidenimino-oxycarbonyl)-pyrimidin vom Schmelzpunkt 188-189 °C.

In entsprechender Weise und gemäß den allgemeinen Angaben zur Herstellung erhält man die folgenden Imidazolinyl-pyrimidine der allgemeinen Formel (I):

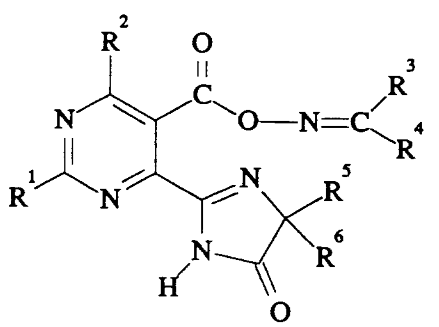

(I)

| Bsp.Nr. | Formel | physikalische Eigenschaften |
|---|---|---|
| 2 | 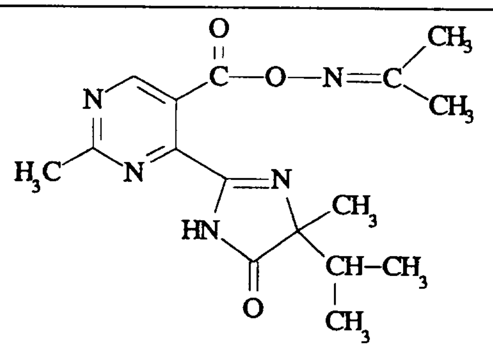 | $^{1}$H-NMR*): 0,82 (d, 3H); 1,02 (d, 3H); 1,35 (s, 3H); 1,99 (s, 3H); 2,07 (s, 3H); 2,83 (s, 3H) |
| 3 | 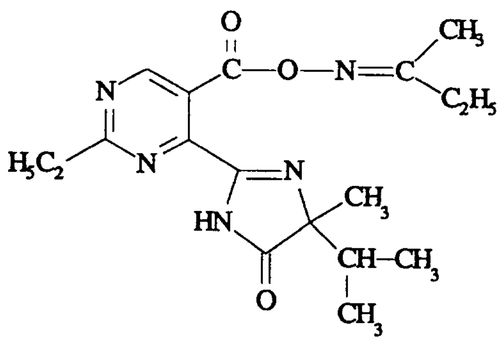 | Fp. 128-129°C |

| Bsp.Nr. | Formel | physikalische Eigenschaften |
|---|---|---|
| 4 | 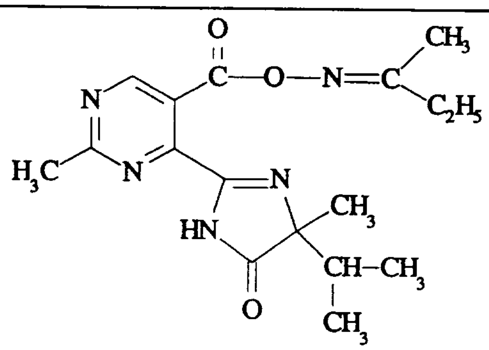 | $^{1}$H-NMR*): 0,81 (d, 3H); 1,02 (d, 3H); 1,34 (s, 3H); 1,96 (s, 3H) |

*) Die $^{1}$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Anwendungsbeispiele:

In den folgenden Anwendungsbeispielen wurde die nachstehend aufgeführte Verbindung als Vergleichssubstanz eingesetzt:

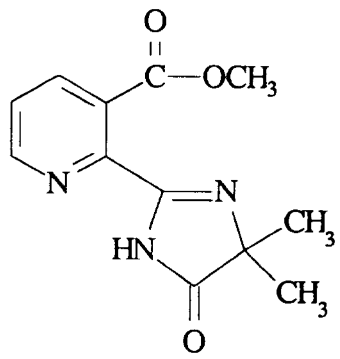

(A)

2-(4,4-Dimethylimidazolin-5-on-2-yl)-pyridin-3-carbonsäuremethylester (bekannt aus EP 41623)

Beispiel A:

**Pre-emergence-Test**

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man ein Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffes pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit bei vergleichbarer Nutzpflanzenselektivität gegenüber dem Stand der Technik zeigt in diesem Test z.B. die Verbindung gemäß Herstellungsbeispiel 1.

Beipiel B:

**Post-emergence-Test**

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man ein Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 bis 15 cm haben so, daß die jeweils gewünschten Wirkstoffmengen pro Flächeneinheit ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100% = totale Vernichtung

Eine deutliche Überlegenheit in der Wirksamkeit bei vergleichbarer Nutzpflanzenselektivität gegenüber dem Stand der Technik zeigt in diesem Test z.B. die Verbindung gemäß Herstellungsbeispiel 1.

## Patentansprüche

1. Neue Imidazolinyl-pyrimidine der allgemeinen Formel (I),

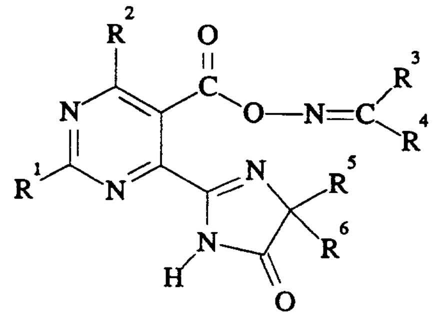

(I)

in welcher

| | |
|---|---|
| $R^1$ | für Wasserstoff, Halogen, Cyano, Nitro oder Thiocyanato oder für einen jeweils gegebenenfalls substitituierten Hydroxy-, Mercapto-, Amino-, Carbonyl-, Sulfinyl-, Sulfonyl-, Alkyl-, Alkenyl-, Alkinyl-, Cycloalkyl-, Cycloalkenyl-, Aryl- oder Heterocyclylrest steht, |
| $R^2$ | für Wasserstoff, Hydroxy, Halogen, Alkyl oder Halogenalkyl steht, |
| $R^3$ | für Wasserstoff oder für Alkyl steht und |
| $R^4$ | für Alkyl oder oder für gegebenenfalls substituiertes Aryl steht oder |
| $R^3$ und $R^4$ | gemeinsam mit dem Kohlenstoffatom, an das sie gebunden sind für einen Cycloalkylrest stehen und |
| $R^5$ und $R^6$ | unabhängig voneinander jeweils für Wasserstoff, Alkyl, Halogenalkyl oder Cycloalkyl stehen. |

2. Imidazolinyl-pyrimidine der allgemeinen Formel (I) gemäß Anspruch 1, dadurch gekennzeichnet, daß

| | |
|---|---|
| $R^1$ | für Wasserstoff, Fluor, Chlor, Brom, Iod, Cyano, Nitro, Hydroxy, Mercapto oder Thiocyanato steht; |
| $R^1$ | außerdem für substituiertes -OR oder -SR steht, wobei als Substituenten für R jeweils infrage kommen: geradkettiges oder verzweigtes Alkyl mit 1 bis 8 Kohlenstoffatomen, geradkettiges oder verzweigtes Halogenalkyl mit 1 bis 8 Kohlenstoffatomen und 1 bis 17 gleichen oder verschiedenen Halogenatomen, geradkettiges oder verzweigtes Alkoxyalkyl mit jeweils 1 bis 8 Kohlenstoffatomen in den einzelnen Alkylteilen, geradkettiges oder verzweigtes Dialkylaminoalkyl mit jeweils 1 bis 8 Kohlenstoffatomen in den einzelnen Alkylteilen, geradkettiges oder verzweigtes Alkanoyl mit 1 bis 9 Kohlenstoffatomen, geradkettiges oder verzweigtes Alkoxycarbonyl mit 2 bis 9 Kohlenstoffatomen, geradkettiges oder verzweigtes Alkylsulfonyl mit 1 bis 8 Kohlenstoffatomen, geradkettiges oder verzweigtes Alkoxysulfonyl mit 1 bis 8 Kohlenstoffatomen, jeweils gegebenenfalls im Aryl- bzw. Heterocyclylteil einfach oder mehrfach, gleich oder verschieden substituiertes Arylalkyl mit 1 bis 8 Kohlenstoffatomen im geradkettigen oder verzweigten Alkylteil und 6 bis 10 Kohlenstoffatomen im Arylteil, Aryl mit 6 bis 10 Kohlenstoffatomen, Arylcarbonyl, Arylsulfonyl oder Aryloxysulfonyl mit jeweils 6 bis 10 Kohlenstoffatomen im Arylteil, Heterocyclyl oder Heterocyclylalkyl mit jeweils 2 bis 9 Kohlenstoffatomen und 1 bis 5 gleichen oder verschiedenen Heteroatomen, insbesondere Stickstoff, Sauerstoff und/oder Schwefel im Heterocyclylteil und gegebenenfalls 1 bis 4 Kohlenstoffatomen im geradkettigen oder verzweigten Alkylteil, wobei als Aryl- bzw. Heterocyclylsubstituenten jeweils infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich |

oder verschieden durch Halogen und/oder geradkettiges oder verzweigtes Alkyl mit 1 bis 4 Kohlenstoffatomen substituiertes Phenyl;

$R^1$ außerdem für gegebenenfalls einfach oder zweifach, gleich oder verschieden substituiertes Amino steht, wobei als Substituenten infrage kommen: geradkettiges oder verzweigtes Alkyl mit 1 bis 8 Kohlenstoffatomen, geradkettiges oder verzweigtes Alkenyl mit 2 bis 8 Kohlenstoffatomen, geradkettiges oder verzweigtes Alkinyl mit 2 bis 8 Kohlenstoffatomen, geradkettiges oder verzweigtes Halogenalkyl mit 1 bis 8 Kohlenstoffatomen und 1 bis 17 gleichen oder verschiedenen Halogenatomen, geradkettiges oder verzweigtes Alkoxyalkyl mit jeweils 1 bis 8 Kohlenstoffatomen in den einzelnen Alkylteilen, geradkettiges oder verzweigtes Dialkylaminoalkyl mit jeweils 1 bis 8 Kohlenstoffatomen in den einzelnen Alkylteilen, geradkettiges oder verzweigtes Alkanoyl mit 1 bis 9 Kohlenstoffatomen, geradkettiges oder verzweigtes Alkoxycarbonyl mit 2 bis 9 Kohlenstoffatomen, geradkettiges oder verzweigtes Alkylsulfonyl mit 1 bis 8 Kohlenstoffatomen, geradkettiges oder verzweigtes Alkoxysulfonyl mit 1 bis 8 Kohlenstoffatomen, geradkettiges oder verzweigtes Dialkylaminosulfonyl mit jeweils 1 bis 8 Kohlenstoffatomen in den einzelnen Alkylteilen, jeweils gegebenenfalls im Aryl- bzw. Heterocyclylteil einfach oder mehrfach, gleich oder verschieden substituiertes Arylalkyl mit 1 bis 8 Kohlenstoffatomen im geradkettigen oder verzweigten Alkylteil und 6 bis 10 Kohlenstoffatomen im Arylteil, Aryl mit 6 bis 10 Kohlenstoffatomen, Arylcarbonyl ,Arylsulfonyl oder Aryloxysulfonyl mit jeweils 6 bis 10 Kohlenstoffatomen im Arylteil, Heterocyclyl oder Heterocyclylalkyl mit jeweils 2 bis 9 Kohlenstoffatomen und 1 bis 5 gleichen oder verschiedenen Heteroatomen, insbesondere Stickstoff, Sauerstoff und/oder Schwefel im Heterocyclylteil und gegebenenfalls 1 bis 4 Kohlenstoffatomen im geradkettigen oder verzweigten Alkylteil, wobei als Aryl- bzw. Heterocyclylsubstituenten jeweils infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;

$R^1$ außerdem für eine jeweils substituierte Carbonyl-, Sulfinyl- oder Sulfonylgruppe steht, wobei als Substituenten jeweils infrage kommen:

Wasserstoff, Hydroxy, Amino, jeweils geradkettiges oder verzweigtes Alkyl, Alkoxy, Alkylamino oder Dialkylamino mit jeweils 1 bis 8 Kohlenstoffatomen in den einzelnen Alkylteilen, jeweils geradkettiges oder verzweigtes Halogenalkyl oder Halogenalkoxy mit jeweils 1 bis 8 Kohlenstoffatomen und 1 bis 17 gleichen oder verschiedenen Halogenatomen, jeweils gegebenenfalls im Arylteil einfach oder mehrfach, gleich oder verschieden substituiertes Aryl, Aryloxy, Arylamino oder Diarylamino mit jeweils 6 bis 10 Kohlenstoffatomen in den einzelnen Arylteilen, wobei als Substituenten in den einzelnen Arylteilen jeweils infrage kommen:

Halogen, Cyano, Nitro, jeweils geradkettiges oder verzweigtes Akyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;

$R^1$ außerdem für gegebenenfalls einfach oder mehrfach, gleich oder verschieden substituiertes, geradkettiges oder verzweigtes Alkyl mit 1 bis 8 Kohlenstoffatomen steht, wobei als Substituenten infrage kommen:

Fluor, Chlor, Brom, Iod, Hydroxy, Cyano, Nitro, Aminocarbonyl, Formamido, jeweils geradkettiges oder verzweigtes Alkoxy, Halogenalkoxy, Alkylthio, Halogenalkylthio, Alkylamino, Dialkylamino, Alkanoyl, N-Alkylaminocarbonyl, N,N-Dialkylaminocarbonyl, Alkanoylamido oder Alkylsulfonyl mit jeweils 1 bis 8 Kohlenstoffatomen in den

einzelnen Alkylteilen und gegebenenfalls 1 bis 17 gleichen oder verschiedenen Halogenatomen, jeweils gegebenenfalls im Aryl- oder Heterocyclylteil einfach oder mehrfach, gleich oder verschieden substituiertes Aryl, Aryloxy, Arylthio, Arylamino, Diarylamino, Arylcarbonyl oder Arylsulfonyl mit jeweils 6 bis 10 Kohlenstoffatomen in den einzelnen Arylteilen oder Heterocyclyl mit 2 bis 9 Kohlenstoffatomen und 1 bis 5 gleichen oder verschiedenen Heteroatomen, insbesondere Stickstoff, Sauerstoff und/oder Schwefel, wobei als Aryl- oder Heterocyclylsubstituenten jeweils infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;

$R^1$ außerdem für gegebenenfalls einfach oder mehrfach, gleich oder verschieden substituiertes, geradkettiges oder verzweigtes Alkenyl mit 2 bis 8 Kohlenstoffatomen steht, wobei als Substituenten infrage kommen:

Fluor, Chlor, Brom, Iod oder Cyano;

$R^1$ außerdem für gegebenenfalls einfach oder mehrfach, gleich oder verschieden substituiertes, geradkettiges oder verzweigtes Alkinyl mit 2 bis 8 Kohlenstoffatomen steht, wobei als Substituenten infrage kommen:

Fluor, Chlor, Brom oder Iod;

$R^1$ außerdem für jeweils gegebenenfalls einfach oder mehrfach, gleich oder verschieden substituiertes Cycloalkyl oder Cycloalkenyl mit jeweils 3 bis 8 Kohlenstoffatomen steht, wobei als Substituenten jeweils infrage kommen:

Fluor, Chlor, Brom, Iod sowie jeweils geradkettiges oder verzweigtes Alkyl oder Halogenalkyl mit jeweils 1 bis 8 Kohlenstoffatomen und gegebenenfalls 1 bis 17 gleichen oder verschiedenen Halogenatomen; außerdem für jeweils gegebenenfalls einfach oder mehrfach, gleich oder verschieden substituiertes Aryl mit 6 bis 10 Kohlenstoffatomen oder Heterocyclyl mit 2 bis 9 Kohlenstoffatomen und 1 bis 5 gleichen oder verschiedenen Heteroatomen, insbesondere Stickstoff, Sauerstoff und/oder Schwefel steht, wobei als Aryl- bzw. Heterocyclylsubstituenten jeweils infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;

$R^2$ für Wasserstoff, Hydroxy, Fluor, Chlor, Brom, Iod, für geradkettiges oder verzweigtes Alkyl mit 1 bis 8 Kohlenstoffatomen oder für geradkettiges oder verzweigtes Halogenalkyl mit 1 bis 8 Kohlenstoffatomen und 1 bis 17 gleichen oder verschiedenen Halogenatomen steht,

$R^3$ für Wasserstoff oder für geradkettiges oder verzweigtes Alkyl mit 1 bis 12 Kohlenstoffatomen steht und

$R^4$ für geradkettiges oder verzweigtes Alkyl mit 1 bis 12 Kohlenstoffatomen oder für gegebenenfalls einfach oder mehrfach, gleich oder verschieden substituiertes Aryl mit 6 bis 10 Kohlenstoffatomen steht, wobei als Arylsubstituenten infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich

oder verschieden durch Halogen und/oder geradkettiges oder verzweigtes Alkyl mit 1 bis 4 Kohlenstoffatomen substituiertes Phenyl oder

$R^3$ und $R^4$ gemeinsam mit dem Kohlenstoffatom, an das sie gebunden sind für einen Cycloalkylrest mit 3 bis 8 Kohlenstoffatomen stehen und

$R^5$ und $R^6$ unabhängig voneinander jeweils für Wasserstoff, für geradkettiges oder verzweigtes Alkyl mit 1 bis 8 Kohlenstoffatomen, für geradkettiges oder verzweigtes Halogenalkyl mit 1 bis 8 Kohlenstoffatomen und 1 bis 17 gleichen oder verschiedenen Halogenatomen oder für Cycloalkyl mit 3 bis 8 Kohlenstoffatomen stehen.

**3.** Imidazolinyl-pyrimidine der allgemeinen Formel (I) gemäß Anspruch 1, dadurch gekennzeichnet, daß

$R^1$ für Wasserstoff, Fluor, Chlor, Brom, Cyano, Nitro, Hydroxy, Mercapto oder Thiocyanato steht;

$R^1$ außerdem für substituiertes -OR oder -SR steht, wobei als Substituenten für R jeweils infrage kommen:

geradkettiges oder verzweigtes Alkyl mit 1 bis 6 Kohlenstoffatomen, geradkettiges oder verzweigtes Halogenalkyl mit 1 bis 6 Kohlenstoffatomen und 1 bis 13 gleichen oder verschiedenen Halogenatomen, geradkettiges oder verzweigtes Alkoxyalkyl mit jeweils 1 bis 6 Kohlenstoffatomen in den einzelnen Alkylteilen, geradkettiges oder verzweigtes Dialkylaminoalkyl mit jeweils 1 bis 6 Kohlenstoffatomen in den einzelnen Alkylteilen, geradkettiges oder verzweigtes Alkanoyl mit 1 bis 7 Kohlenstoffatomen, geradkettiges oder verzweigtes Alkoxycarbonyl mit 2 bis 7 Kohlenstoffatomen, geradkettiges oder verzweigtes Alkylsulfonyl mit 1 bis 6 Kohlenstoffatomen, geradkettiges oder verzweigtes Alkoxysulfonyl mit 1 bis 6 Kohlenstoffatomen, jeweils gegebenenfalls im Aryl- bzw. Heterocyclylteil einfach bis dreifach, gleich oder verschieden substituiertes Arylalkyl mit 1 bis 6 Kohlenstoffatomen im geradkettigen oder verzweigten Alkylteil und 6 oder 10 Kohlenstoffatomen im Arylteil, Aryl mit 6 oder 10 Kohlenstoffatomen, Arylcarbonyl, Arylsulfonyl oder Aryloxysulfonyl mit jeweils 6 oder 10 Kohlenstoffatomen im Arylteil, Heterocyclyl oder Heterocyclylalkyl mit jeweils 2 bis 9 Kohlenstoffatomen und 1 bis 4 gleichen oder verschiedenen Heteroatomen, insbesondere Stickstoff, Sauerstoff und/oder Schwefel im Heterocyclylteil und gegebenenfalls 1 bis 4 Kohlenstoffatomen im geradkettigen oder verzweigten Alkylteil, wobei als Aryl- bzw. Heterocyclylsubstituenten jeweils infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;

$R^1$ außerdem für gegebenenfalls einfach oder zweifach, gleich oder verschieden substituiertes Amino steht, wobei als Substituenten infrage kommen:

geradkettiges oder verzweigtes Alkyl mit 1 bis 6 Kohlenstoffatomen, geradkettiges oder verzweigtes Alkenyl mit 2 bis 6 Kohlenstoffatomen, geradkettiges oder verzweigtes Alkinyl mit 2 bis 6 Kohlenstoffatomen, geradkettiges oder verzweigtes Halogenalkyl mit 1 bis 6 Kohlenstoffatomen und 1 bis 13 gleichen oder verschiedenen Halogenatomen, geradkettiges oder verzweigtes Alkoxyalkyl mit jeweils 1 bis 6 Kohlenstoffatomen in den einzelnen Alkylteilen, geradkettiges oder verzweigtes Dialkylaminoalkyl mit jeweils 1 bis 6 Kohlenstoffatomen in den einzelnen Alkylteilen, geradkettiges oder verzweigtes Alkanoyl mit 1 bis 7 Kohlenstoffatomen, geradkettiges oder verzweigtes Alkoxycarbonyl mit 2 bis 7 Kohlenstoffatomen, geradkettiges oder verzweigtes Alkylsulfonyl mit 1 bis 6 Kohlenstoffatomen, geradkettiges oder verzweigtes Alkoxysulfonyl mit 1 bis 6 Kohlenstoffatomen, geradkettiges oder verzweigtes Dialkylaminosulfonyl mit jeweils 1 bis 6 Kohlenstoffatomen in den einzelnen Alkylteilen, jeweils gegebenenfalls im Aryl- bzw. Heterocyclylteil einfach bis dreifach, gleich oder verschieden substituiertes Arylalkyl mit 1 bis 6 Kohlenstoffatomen im geradkettigen oder verzweigten Alkylteil und 6 oder 10 Kohlenstoffatomen im Arylteil, Aryl mit 6 bis 10 Kohlenstoffatomen, Arylcarbonyl ,Arylsulfonyl oder Aryloxysulfonyl mit jeweils

6 oder 10 Kohlenstoffatomen im Arylteil, Heterocyclyl oder Heterocyclylalkyl mit jeweils 2 bis 9 Kohlenstoffatomen und 1 bis 4 gleichen oder verschiedenen Heteroatomen, insbesondere Stickstoff, Sauerstoff und/oder Schwefel im Heterocyclylteil und gegebenenfalls 1 bis 4 Kohlenstoffatomen im geradkettigen oder verzweigten Alkylteil, wobei als Aryl- bzw. Heterocyclylsubstituenten jeweils infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;

$R^1$ außerdem für eine jeweils substituierte Carbonyl-, Sulfinyl- oder Sulfonylgruppe steht, wobei als Substituenten jeweils infrage kommen:

Wasserstoff, Hydroxy, Amino, jeweils geradkettiges oder verzweigtes Alkyl, Alkoxy, Alkylamino oder Dialkylamino mit jeweils 1 bis 6 Kohlenstoffatomen in den einzelnen Alkylteilen, jeweils geradkettiges oder verzweigtes Halogenalkyl oder Halogenalkoxy mit jeweils 1 bis 6 Kohlenstoffatomen und 1 bis 13 gleichen oder verschiedenen Halogenatomen, jeweils gegebenenfalls im Arylteil einfach bis dreifach, gleich oder verschieden substituiertes Aryl, Aryloxy, Arylamino oder Diarylamino mit jeweils 6 oder 10 Kohlenstoffatomen in den einzelnen Arylteilen, wobei als Substituenten in den einzelnen Arylteilen jeweils infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;

$R^1$ außerdem für gegebenenfalls einfach bis fünffach, gleich oder verschieden substituiertes, geradkettiges oder verzweigtes Alkyl mit 1 bis 6 Kohlenstoffatomen steht, wobei als Substituenten infrage kommen:

Fluor, Chlor, Brom, Iod, Hydroxy, Cyano, Nitro, Aminocarbonyl, Formamido, jeweils geradkettiges oder verzweigtes Alkoxy, Halogenalkoxy, Alkylthio, Halogenalkylthio, Alkylamino, Dialkylamino, Alkanoyl, N-Alkylaminocarbonyl, N,N-Dialkylaminocarbonyl, Alkanoylamido oder Alkylsulfonyl mit jeweils 1 bis 6 Kohlenstoffatomen in den einzelnen Alkylteilen und gegebenenfalls 1 bis 13 gleichen oder verschiedenen Halogenatomen, jeweils gegebenenfalls im Aryl- oder Heterocyclylteil einfach bis dreifach, gleich oder verschieden substituiertes Aryl, Aryloxy, Arylthio, Arylamino, Diarylamino, Arylcarbonyl oder Arylsulfonyl mit jeweils 6 oder 10 Kohlenstoffatomen in den einzelnen Arylteilen oder Heterocyclyl mit 2 bis 9 Kohlenstoffatomen und 1 bis 4 gleichen oder verschiedenen Heteroatomen, insbesondere Stickstoff, Sauerstoff und/oder Schwefel, wobei als Aryl- oder Heterocyclylsubstituenten jeweils infrage kommen:

Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;

$R^1$ außerdem für gegebenenfalls einfach bis fünffach, gleich oder verschieden substituiertes, geradkettiges oder verzweigtes Alkenyl mit 2 bis 6 Kohlenstoffatomen steht, wobei als Substituenten infrage kommen:

Fluor, Chlor, Brom oder Cyano;

$R^1$ außerdem für gegebenenfalls einfach bis dreifach, gleich oder verschieden substituiertes, geradkettiges oder verzweigtes Alkinyl mit 2 bis 6 Kohlenstoffatomen steht, wobei als Substituenten infrage kommen:
Fluor, Chlor oder Brom;

$R^1$ außerdem für jeweils gegebenenfalls einfach bis fünffach, gleich oder verschieden substituiertes Cycloalkyl oder Cycloalkenyl mit jeweils 3 bis 7 Kohlenstoffatomen steht, wobei als Substituenten jeweils infrage kommen:
Fluor, Chlor, Brom, Iod sowie jeweils geradkettiges oder verzweigtes Alkyl oder Halogenalkyl mit jeweils 1 bis 6 Kohlenstoffatomen und gegebenenfalls 1 bis 13 gleichen oder verschiedenen Halogenatomen;

$R^1$ außerdem für jeweils gegebenenfalls einfach bis fünffach, gleich oder verschieden substituiertes Aryl mit 6 oder 10 Kohlenstoffatomen oder Heterocyclyl mit 2 bis 9 Kohlenstoffatomen und 1 bis 4 gleichen oder verschiedenen Heteroatomen, insbesondere Stickstoff, Sauerstoff und/oder Schwefel steht, wobei als Aryl- bzw. Heterocyclylsubstituenten jeweils infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl;

$R^2$ für Wasserstoff, Hydroxy, Fluor, Chlor, Brom, Iod, für geradkettiges oder verzweigtes Alkyl mit 1 bis 6 Kohlenstoffatomen oder für geradkettiges oder verzweigtes Halogenalkyl mit 1 bis 6 Kohlenstoffatomen und 1 bis 13 gleichen oder verschiedenen Halogenatomen steht,

$R^3$ für Wasserstoff oder für geradkettiges oder verzweigtes Alkyl mit 1 bis 8 Kohlenstoffatomen steht und

$R^4$ für geradkettiges oder verzweigtes Alkyl mit 1 bis 8 Kohlenstoffatomen oder für gegebenenfalls einfach bis fünffach, gleich oder verschieden substituiertes Aryl mit 6 oder 10 Kohlenstoffatomen steht, wobei als Arylsubstituenten infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl oder

$R^3$ und $R^4$ gemeinsam mit dem Kohlenstoffatom, an das sie gebunden sind für einen Cycloalkylrest mit 3 bis 7 Kohlenstoffatomen stehen und

$R^5$ und $R^6$ unabhängig voneinander jeweils für Wasserstoff, für geradkettiges oder verzweigtes Alkyl mit 1 bis 6 Kohlenstoffatomen, für geradkettiges oder verzweigtes Halogenalkyl mit 1 bis 6 Kohlenstoffatomen und 1 bis 13 gleichen oder verschiedenen Halogenatomen oder für Cycloalkyl mit 3 bis 7 Kohlenstoffatomen stehen.

**4.** Imidazolinyl-pyrimidine der allgemeinen Formel (I) gemäß Anspruch 1, dadurch gekennzeichnet, daß

$R^1$ für Wasserstoff, Hydroxy oder Mercapto steht;

$R^1$ außerdem für substituiertes -OR oder -SR steht, wobei als Substituenten jeweils infrage kommen:
geradkettiges oder verzweigtes Alkyl mit 1 bis 4 Kohlenstoffatomen, geradkettiges oder verzweigtes Alkoxyalkyl mit jeweils 1 bis 4 Kohlenstoffatomen in den einzelnen Alkylteilen, geradkettiges oder verzweigtes Dialkylaminoalkyl mit jeweils 1 bis 4 Kohlenstoffatomen in den einzelnen Alkylteilen, geradkettiges oder verzweigtes Halogenalkyl mit 1 bis 4 Kohlenstoffatomen und 1 bis 9 gleichen oder verschiedenen Halogenatomen oder jeweils gegebenenfalls im Phenyl- bzw. Heteroarylteil einfach bis dreifach, gleich oder verschieden substituiertes Phenylalkyl mit 1 bis 4 Kohlenstoffatomen im geradkettigen oder verzweigten Alkylteil, Phenyl, Pyridyl, Pyridylmethyl, Furanylmethyl, Thienylmethyl, Thiazolylmethyl oder Triazolylmethyl, wobei als Phenyl- bzw. Heteroarylsubstituenten jeweils infrage kommen:
Fluor, Chlor, Brom, Iod, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen - insbesondere Fluor, Chlor, Brom und/oder Iod;

$R^1$ außerdem für gegebenenfalls einfach oder zweifach, gleich oder verschieden substituiertes Amino steht, wobei als Substituenten infrage kommen:
geradkettiges oder verzweigtes Alkyl mit 1 bis 4 Kohlenstoffatomen, geradkettiges oder verzweigtes Alkenyl mit 2 bis 6 Kohlenstoffatomen, Allyl, Propargyl, geradkettiges oder verzweigtes Alkoxyalkyl mit jeweils 1 bis 4 Kohlenstoffatomen in den einzelnen Alkylteilen, geradkettiges oder verzweigtes Dialkylaminoalkyl mit jeweils 1 bis 4 Kohlenstoffatomen in den einzelnen Alkylteilen, geradkettiges oder verzweigtes Alkanoyl mit 1 bis 5 Kohlenstoffatomen, geradkettiges oder verzweigtes Alkoxycarbonyl mit 2 bis 5 Kohlenstoffatomen, geradkettiges oder verzweigtes Alkylsulfonyl mit 1 bis 4 Kohlenstoffatomen;

$R^1$ außerdem für eine jeweils substituierte Carbonyl-, Sulfinyl- oder Sulfonylgruppe steht, wobei als Substituenten jeweils infrage kommen:
jeweils geradkettiges oder verzweigtes Alkyl, Alkoxy, Alkylamino oder Dialkylamino mit jeweils 1 bis 4 Kohlenstoffatomen in den einzelnen Alkylteilen;

$R^1$ außerdem für gegebenenfalls einfach bis fünffach, gleich oder verschieden substituiertes, geradkettiges oder verzweigtes Alkyl mit 1 bis 6 Kohlenstoffatomen steht, wobei als Substituenten infrage kommen:
Fluor, Chlor, jeweils geradkettiges oder verzweigtes Alkoxy oder Alkylthio mit jeweils 1 bis 4 Kohlenstoffatomen; geradkettiges oder verzweigtes Halogenalkoxy oder Halogenalkylthio mit jeweils 1 bis 4 Kohlenstoffatomen und 1 bis 9 gleichen oder verschiedenen Halogenatomen - insbesondere Fluor, Chlor und/oder Brom - oder gegebenenfalls einfach bis dreifach, gleich oder verschieden substituiertes Phenyl, wobei als Substituenten jeweils infrage kommen:
Halogen, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen;

$R^1$ außerdem für geradkettiges oder verzweigtes Alkenyl mit 2 bis 6 Kohlenstoffatomen steht;

$R^1$ außerdem für geradkettiges oder verzweigtes Alkinyl mit 2 bis 6 Kohlenstoffatomen steht;

$R^1$ außerdem für gegebenenfalls einfach bis dreifach, gleich oder verschieden durch Methyl und/oder Chlor substituiertes Cyclopropyl steht;

$R^1$ außerdem für gegebenenfalls einfach bis dreifach, gleich oder verschieden substituiertes Phenyl steht, wobei als Substituenten jeweils infrage kommen:
Fluor, Chlor, Brom, Iod, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen - insbesondere Fluor, Chlor, Brom und/oder Iod;

$R^1$ außerdem für jeweils gegebenenfalls einfach bis dreifach, gleich oder verschieden substituiertes Pyridyl, Furanyl, Tetrahydrofuranyl, Thienyl oder Thiazolyl steht, wobei als Substituenten jeweils infrage kommen:
jeweils geradkettiges oder verzweigtes Alkyl oder Alkoxy mit jeweils 1 bis 4 Kohlenstoffatomen;

$R^2$ für Wasserstoff oder Methyl steht;

$R^3$ für Wasserstoff oder für geradkettiges oder verzweigtes Alkyl mit 1 bis 6 Kohlenstoffatomen steht und

$R^4$ für geradkettiges oder verzweigtes Alkyl mit 1 bis 6 Kohlenstoffatomen oder für gegebenenfalls einfach bis dreifach, gleich oder verschieden substituiertes Phenyl

steht, wobei als Phenylsubstituenten infrage kommen:
Fluor, Chlor, Brom, Iod, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy, Halogenalkylthio mit jeweils 1 bis 4 Kohlenstoffatomen und 1 bis 9 gleichen oder verschiedenen Halogenatomen - insbesondere Fluor, Chlor, Brom und/oder Iod oder

$R^3$ und $R^4$ gemeinsam mit dem Kohlenstoffatom, an das sie gebunden sind für einen Cycloalkylrest mit 3 bis 6 Kohlenstoffatomen stehen,

$R^5$ für Wasserstoff, Methyl oder Fluormethyl steht und

$R^6$ für Methyl, Ethyl, n- oder i-Propyl oder Cyclopropyl steht.

**5.** Verfahren zur Herstellung von neuen Imidazolinyl-pyrimidinen der allgemeinen Formel (I),

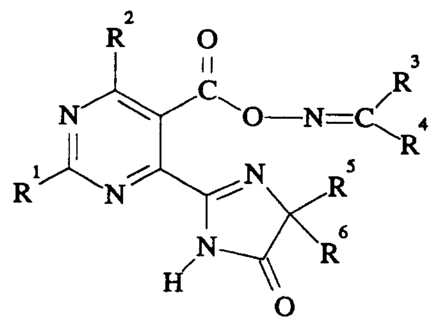

(I)

in welcher

$R^1$ für Wasserstoff, Halogen, Cyano, Nitro oder Thiocyanato oder für einen jeweils gegebenenfalls substitituierten Hydroxy-, Mercapto-, Amino-, Carbonyl-, Sulfinyl-, Sulfonyl-, Alkyl-, Alkenyl-, Alkinyl-, Cycloalkyl-, Cycloalkenyl-, Aryl- oder Heterocyclylrest steht,

$R^2$ für Wasserstoff, Hydroxy, Halogen, Alkyl oder Halogenalkyl steht,

$R^3$ für Wasserstoff oder für Alkyl steht und

$R^4$ für Alkyl oder für gegebenenfalls substituiertes Aryl steht oder

$R^3$ und $R^4$ gemeinsam mit dem Kohlenstoffatom , an das sie gebunden sind für einen Cycloalkylrest stehen und

$R^5$ und $R^6$ unabhängig voneinander jeweils für Wasserstoff, Alkyl, Halogenalkyl oder Cycloalkyl stehen,

dadurch gekennzeichnet daß man Pyrimidin-dicarbonsäurediester der Formel (II),

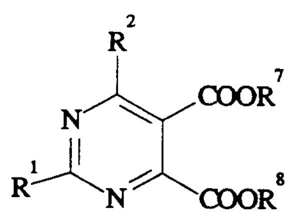

(II)

in welcher

$R^1$ und $R^2$ die oben angegebenen Bedeutungen haben und

$R^7$ und $R^8$ unabhängig voneinander jeweils für Alkyl stehen,

mit Aminosäureamiden der Formel (III),

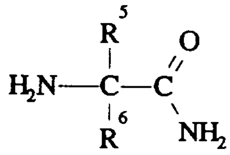

(III)

in welcher
$R^5$ und $R^6$ die oben angegebenen Bedeutungen haben,
zunächst in einer ersten Stufe gegebenenfalls in Gegenwart eines Verdünnungsmittels und gegebenenfalls in Gegenwart eines Reaktionshilfsmittels umsetzt und in einer anschließenden Folgereaktion die so erhältlichen Imidazolinylpyrimidin-carbonsäuren der Formel (IV),

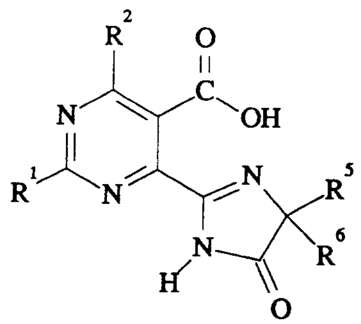

(IV)

in welcher
$R^{1,}$ $R^2$, $R^5$ und $R^6$ die oben angegebenen Bedeutungen haben,
zunächst in einer 2.Stufe mit einem Kondensationsmittel gegebenenfalls in Gegenwart eines Verdünnungsmittels und gegebenenfalls in Gegenwart eines Reaktionshilfsmittels umsetzt und anschließend die so erhältlichen Imidazo-pyrrolo-pyrimidine der Formeln (Va) und (Vb),

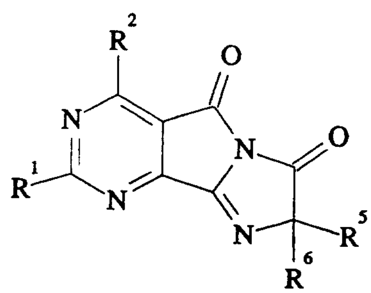

(Va)

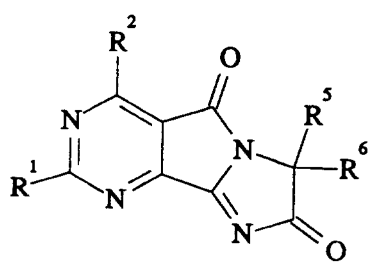

(Vb)

in welche n
$R^1$, $R^2$, $R^5$ und $R^6$ die oben angegebenen Bedeutungen haben,
in einer anschließenden 3. Stufe mit Oximen der Formel (VI),

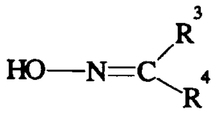

(VI)

in welcher

$R^3$ und $R^4$ die oben angegebenen Bedeutungen haben,

gegebenenfalls in Gegenwart eines Verdünnungsmittels und gegebenenfalls in Gegenwart eines Reaktionshilfsmittels umsetzt.

**6.** Verfahren zur Bekämpfung von unerwünschten Pflanzen, dadurch gekennzeichnet, daß man Imidazolinyl-pyrimidine der allgemeinen Formel (I) gemäß den Ansprüchen 1 bis 5 auf unerwünschte Pflanzen und/oder ihren Lebensraum einwirken läßt.

**7.** Verwendung von Imidazolinyl-pyrimidinen der allgemeinen Formel (I) gemäß den Ansprüchen 1 bis 5 zur Bekämpfung von unerwünschten Pflanzen.

**8.** Verfahren zur Herstellung von herbiziden Mitteln, dadurch gekennzeichnet, daß man Imidazolinyl-pyrimidine der allgemeinen Formel (I) gemäß den Ansprüchen 1 bis 5 mit Streckmitteln und/oder oberflächenaktiven Substanzen vermischt

**9.** Herbizide Mittel, gekennzeichnet durch einen Gehalt an mindestens einem Imidazolinyl-pyrimidin der allgemeinen Formel (I).

Europäisches Patentamt

# EUROPÄISCHER RECHERCHENBERICHT

Nummer der Anmeldung
EP 93 11 5090

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| P,A | EP-A-0 517 052 (BAYER) 9. Dezember 1992<br>* das ganze Dokument *<br>----- | 1-9 | C07D403/04<br>C07D487/14<br>A01N43/58 |

| RECHERCHIERTE SACHGEBIETE (Int.Cl.5) |
|---|
| C07D |

Der vorliegende Recherchenbericht wurde für alle Patentansprüche erstellt

| Recherchenort | Abschlußdatum der Recherche | Prüfer |
|---|---|---|
| DEN HAAG | 15. Februar 1994 | Francois, J |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
........................................
& : Mitglied der gleichen Patentfamilie, übereinstimmendes Dokument